(19) Europäisches Patentamt
European Patent Office
Office européen des brevets

(11) Publication number: **0 024 091**
**B1**

(12) **EUROPEAN PATENT SPECIFICATION**

(45) Date of publication of patent specification: **03.04.85**

(51) Int. Cl.[4]: **C 07 C 45/50,** C 07 C 27/20, B 01 J 31/02, B 01 J 31/12, C 07 F 7/08, C 07 F 9/50

(21) Application number: **80900540.8**

(22) Date of filing: **12.02.80**

(86) International application number: **PCT/US80/00213**

(87) International publication number: **WO 80/01690 21.08.80 Gazette 80/19**

# (54) HYDROFORMYLATION PROCESS AND NOVEL TRANSITION METAL CATALYSTS.

(30) Priority: **12.02.79 US 11238**
**29.05.79 US 43548**
**23.01.80 US 114627**

(43) Date of publication of application: **25.02.81 Bulletin 81/08**

(45) Publication of the grant of the patent: **03.04.85 Bulletin 85/14**

(84) Designated Contracting States: **FR**

(56) References cited:
**US-A-3 501 403**
**US-A-3 527 809**
**US-A-3 832 404**
**US-A-3 859 359**
**US-A-3 890 359**
**US-A-3 907 852**
**US-A-3 939 188**
**US-A-3 948 965**

**The file contains technical information submitted after the application was filed and not included in this specification**

(73) Proprietor: **Exxon Research and Engineering Company**
**P.O.Box 390 180 Park Avenue**
**Florham Park New Jersey 07932 (US)**

(72) Inventor: **OSWALD, Alexis A.**
**1098 Sunny Slope Drive**
**Mountainside, NJ 07091 (US)**
Inventor: **JERMANSEN, Torris G.**
**245 Getz Avenue**
**Staten Island, NY 10312 (US)**
Inventor: **WESTNER, Andrew A.**
**1 Short Way**
**Paramus, NJ 07652 (US)**
Inventor: **HUANG, I-Der**
**Rifle Camp Road**
**West Paterson, NJ 07424 (US)**

(74) Representative: **Pitkin, Robert Wilfred et al**
**ESSO Engineering (Europe) Ltd. Patents & Licences Apex Tower High Street**
**New Malden Surrey KT3 4DJ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(56) References cited:
US-A-3 954 877
US-A-4 083 803
US-A-4 098 727
US-A-4 107 079
US-A-4 134 906
US-A-4 144 191
US-A-4 151 114
US-A-4 179 401

## Description

The present invention is related to selective alkyl diaryl phosphine transition metal complex olefin hydroformylation catalysts. More particularly, the subject of this invention is certain, new selective alkyl diaryl phosphine rhodium complex hydroformylation catalysts. As such, the invention is specifically related to substituted tris-(alkyl diphenyl phosphine) rhodium carbonyl hydride complexes.

A special aspect of the invention is concerned with the preparation, physicochemical and catalytic properties of the novel complexes, i.e., phosphine basicity, stereochemistry versus complex formation, thermal stability of complexes, correlations of catalyst selectivity and activity with hydroformylation reaction rates at different temperatures. The effects of excess phosphine ligand, excess hydrogen, carbon monoxide partial pressure on the catalytic properties and on catalyst selectivity to linear aldehydes are also related.

Transition metal complexes containing phosphine ligands have been widely studied as catalysts for hydroformylation and hydrogenation. General application of such complexes in reaction with carbon monoxide are discussed, e.g., in the monograph of Juergen Falbe, "Carbon Monoxide in Organic Synthesis, Springer Verlag, New York, 1970.

There were a number of all inclusive patent disclosures on the use of phosphine rhodium complexes as hydroformylation catalysts: German Offenlegungsschrift 2,758,473 by W. E. Smith (assigned to General Electric) disclosed them for allyl alcohol hydroformylation; U.S. Patent No. 4,137,240 by M. L. Peterson (assigned to E. I. DuPont de Nemours and Co.) described them for 2-vinyl-4-methyl-1,3-dioxane batch hydroformylation; U.S. Patent No. 3,965,192 by F. B. Booth (assigned to Union Oil Co. of California) disclosed them for the hydroformylation of monoolefins; U.S. Patent No. 4,041,082 by T. Onoda and T. Masuyama (assigned to Mitsubishi Chemical Industries) defined such complexes broadly in a process for their reactivation; U.S. Patent No. 3,821,311 by O. R. Hughes and M. E. D. Millman (assigned to Celanese Corp.) also disclosed the use of such complexes broadly when used with bases for combined hydroformylation, aldolization. Similarly, British Patent No. 1,243,189 by M. J. Lawrenson and G. Foster (assigned to British Petroleum Co., Ltd.) provided an all inclusive definition of phosphines in such catalyst complexes also containing chelating diketones. Finally, U.S. Patent No. 4,052,461 by H. B. Tinker and D. E. Morris (assigned to Monsanto Co.) disclosed rhodium containing cations which can include any tertiary phosphine.

Most of the prior art work was carried out with either triaryl phosphine or trialkyl phosphine complexes. The present study concentrated on an investigation of the complexes of some "mixed ligand structures," i.e., alkyl diphenyl phosphines. Prior to the present work, rhodium complexes of these ligands could not be used to advantage.

The basic chemistry of hydroformylation and its catalysis by transition metal compounds, including phosphine-rhodium complexes is known and has been recently reviewed and summarized by R. L. Pruett in Vol. 17 of "Advances in Organo Metallic Chemistry" edited by S. G. Stone and R. West, *Academic Press*, New York, New York 1979 in a chapter entitled "Hydroformylation", Pruett concluded that, for a selective rhodium catalyzed hydroformylation of alpha-olefins to n-aldehydes, critical combinations of several reaction parameters were required. The author states that these parameters included low partial pressure of carbon monoxide, high concentration of excess phosphite or aryl phosphine ligands and low total gas pressure.

In U.S. Patent Nos. 3,527,809 and 3,917,661; Pruett and Smith state that suitable ligands for rhodium catalysts for hydroformylation must be weakly basic, having a half neutralization potential (Δ HNP) of at least 425 millivolts (preferably 500) above that of diphenyl guanidine. As such weakly basic ligands, Pruett and Smith mentioned among others phosphites and triaryl phosphines. They specifically indicate that stronger phosphine bases, such as diaryl alkyl phosphines, should be excluded as ligands for selective rhodium catalysis. Similar disclosures are also contained in Pruett and Smith's U.S. Patent No. 4,148,830, where they additionally state that suitable ligands should be free of sterically hindered aromatic groups.

In German Offenlegungsschrift 2,802,922 (based on U.S. Serial No. 762,335, filed on January 25, 1977 in the names of D. G. Morrell and P. D. Sherman, Jr.), there is described a process including the addition of small amounts of diaryl alkyl phosphine ligands to a tris-triphenyl phosphine rhodium complex system. However, substantially all of the free ligand in the Morrell et al. system is a triaryl phosphine ligand, and it is specifically stated that the invention is not intended to include the use of diaryl alkyl phosphine ligands alone. Some of the diaryl alkyl phosphine ligands which are apparently disclosed in this German publication for use in that particular content include methyl diphenyl phosphine, ethyl diphenyl phosphine, propyl diphenyl phosphine, butyl diphenyl phosphine, ethyl-bis(p-methoxy phenyl) phosphine, ethylphenyl-p-biphenyl phosphine, methyl-phenyl-p(N,N-dimethylaminophenyl) phosphine, propyl-phenyl-p-(N,N-dimethylaminophenyl) phosphine, and propyl-bis-(p-methoxy phenyl) phosphine.

Still other patents and publications also mention the use of certain other diaryl alkyl phosphines as ligands in rhodium catalyzed hydroformylation reactions. For example, ethyl ditolyl phosphine is mentioned as a possible ligand by Peterson in U.S. Patent No. 4,137,240. Wilkinson, U.S. Patent No. 4,108,905, discloses ethyl diphenyl phosphine as a ligand for a rhodium hydrido carbonyl complex, which he says may be used in the presence of molten triphenyl phosphine as reaction medium. British Patent No. 2,014,138 discloses the use of, among others, alkyl diaryl phosphines, e.g., propyl diphenyl phosphine, in

combination with certain diphosphino alkanes in rhodium hydrido carbonyl complex systems. Booth in U.S. Patent No. 3,560,539 mentions as a ligand ethyl diphenyl phosphine, while Booth et al., U.S. Patent No. 3,644,446, discloses as possible ligands ethyl diphenyl phosphine and methyl dixylyl phosphine, Slaugh et al., in U.S. Patent No. 3,239,566 mentions diphenyl butyl phosphine, methyl diphenyl phosphine, ethyl diphenyl phosphine and diphenyl benzyl phosphine as possible ligands for rhodium or ruthenium catalysts. *Chemistry Letters*, (1972) pp. 483—488, refers to a rhodium complex bonded to (+)-diphenylneomenthyl phosphine.

Other Union Carbide researchers disclosed additional inventions mostly related to the commerical TPP-rhodium complex catalyzed process. German Offenlegungsschrift 2,715,685 by E. A. V. Brewster and R. L. Pruett described the continuous process in detail. Also, it showed the harmful effect of aldehydes having conjugated olefinic unsaturation. German Offenlegungsschrift 2,730,527 by R. W. Halstead and J. C. Chaty disclosed the addition of appropriate, minor amounts of oxygen to the reaction mixture of the continuous process to maintain activity.

Alkyl diaryl phosphine ligands were specifically disclosed as potential rhodium catalyst stabilizer ligands in a number of patents and journal articles on rhodium catalyzed hydroformylation. U.S. Patent No. 4,108,905 by G. Wilkinson (assigned to Johnson Matthey & Co., Ltd.) disclosed ethyl diphenyl phosphine as a stabilizing ligand as a part of an all inclusive, but sparsely supported, disclosure on phosphine ligands. British Patent Application No. 2,014,138 (assigned to Kuraray Co., Ltd.) similarly disclosed bis-diarylphosphino alkanes, i.e., diaryl phosphine substituted alkyl diaryl phosphines, as stabilizing ligands. U.S. Patent No. 4,151,209 by J. L. Paul, W. L. Pieper and L. W. Wade (assigned to Celanese Corp.) reported on the formation of propyl diphenyl phosphine ligand from the TPP-rhodium catalyst during propylene hydroformylation. U.S. Patent Nos. 3,560,539; 3,644,446 and 3,801,646 by F. B. Booth (assigned to Union Oil Co. of California) disclosed the derivation of undefined rhodium catalyst complexes by reduction, starting with a variety of phosphines including methyl diphenyl phosphine or propyl diphenyl phosphine. U.S. Patent No. 3,239,566 by L. H. Slaugh and R. D. Mullineaux (assigned to Shell Oil Co.) disclosed methyl diphenyl phosphine, ethyl diphenyl phosphine and benzyl diphenyl phosphine as examples for an all inclusive definition of phosphine complexes of rhodium and ruthenium. Slaugh preferred the complexes of tributyl phosphine, started with rhodium chloride and emphasized the formation of alcohols as well as aldehydes in his process.

There are a number of patents which disclosed asymmetrical, optically active alkyl diaryl phosphines for the stereoselective hydroformylation of special olefins such as styrene, e.g., Canadian Patent No. 1,027,141 by H. B. Tinker and A. J. Solodar (assigned to Monsanto Co.); British Patent No. 1,402,832 by C. Botteghi, G. Consiglio and C. Salomon (assigned to P. Pino); U.S. Patent No. 4,139,565 by J. D. Unruh and L. E. Wade (assigned to Celanese Corp.) and French Patent No. 72,43479 by R. Stern, D. Commereuc, Y. Chavin and H. B. Kagan (assigned to the Institute Francais du Petrole, des Carburants et Lubrifiants). Although these ligands are structurally related to those of the present work, their properties and application is outside the scope of the present invention.

The most conclusive study regarding the effect on hydroformylation catalysis of an excess of a simple alkyl diphenyl phosphine, i.e., ethyl diphenyl phosphine, was published by A. R. Sanger in the Journal of Molecular Catalysis [*3*, pages 221—226, particularly page 222 (1977/1978)]. He reported that the addition of ethyl diphenyl phosphine to the TPP-rhodium catalyst resulted in less increase in catalyst activity at 20°C. than that of excess TPP. He found similar effects when chelating di-alpha,ω-diphenylphosphino-alkanes were added. Using more than molar amounts of 1,4-diphenylphosphino-butane resulted in decreased catalyst activity.

There is much less information on hydroformylation catalysis by the rhodium complexes of substituted aliphatic phosphines, particularly substituted alkyl diaryl phosphines. Catalyst complexes of such phosphines are usually within the all inclusive scope of several patent applications already discussed. However, very few specific disclosures were made. In effect, no direct disclosure of any tris-(substituted alkyl diphenyl phosphine) rhodium carbonyl hydride was found prior to this invention.

In the area of trihydrocarbylsilyl substituted diphenyl phosphine rhodium complexes containing halogen, there are several disclosures by G. Chandra (British Patent Nos. 1,419,769; 1,420,928 and 1,421,136, assigned to Dow Corning Ltd.). Tris-(trimethylsilyl-methyl diphenyl phosphine) rhodium carbonyl chloride is specifically disclosed. Relatively non-selective hydroformylation catalysis by this and similar complexes was recently reported by M. O. Farrell, C. H. Van Dyke, L. J. Boucher and S. J. Metlin [J. Organomet, Chem., *169* (2) 199 (1979)].

Carboxy substituted t-phosphine rhodium and cobalt complexes of rhodium were disclosed in an all inclusive unspecified manner as hydroformylation catalysts in British Patent No. 1,350,822 by BASF A. G. 2-Carboxyethyl diphenyl phosphine was disclosed as an exemplary phosphine ligand.

Halogen, aryloxy, alkoxy, hydroxy, nitro and phenyl substituted phosphine rhodium complexes were included in an all inclusive definition of phosphine rhodium complex hydroformylation catalysts in British Patent No. 1,298,331 by G. Wilkinson (assigned to Johnson, Matthey & Co., Ltd.). However, not a singly substituted alkyl diaryl phosphine compound was named. Similarly, amino, halo and alkyl substituted rhodium complex hydroformylation catalysts were generically disclosed by F. B. Booth in U.S. Patent No, 3,965,192 (assigned to Union Oil Co. of California) which was already referred to. Again, no example of substituted alkyl diaryl phosphine was given.

As far as alkyl diphenyl phosphines are concerned, many compounds are known. However, few aryl or nonhydrocarbyl substituted compounds were disclosed. A complete list of characterized compounds and their preparation, up to 1969, is given in Volume 1, Chapter 1, pages 154 to 162 by L. Maier, as a part of the series of monographs, entitled "Organic Phosphorus Compounds" by G. M. Kosolapoff and L. Maier, J. Wiley & Sons, Inc., New York, New York, 1972. However, none of the heteroorganic substituted compounds of the present invention is disclosed. Chapter 3 by G. Booth of the same book also lists characterized phosphine metal complexes. However, no rhodium carbonyl hydrides are found.

With regard to the synthesis of alkyl diphenyl phosphines in general, Kosolapoff and Maier lists a number of displacement reactions as being frequently used (see page 2). However, there is little information on diphenyl phosphine to olefin additions. No substituted alkyl diphenyl phosphine derived via addition is disclosed. As far as the hydrido carbonyl rhodium complexes of phosphines are concerned, the known, obviously applicable syntheses, are reviewed in Booth's chapter. They do not include the presently recommended methods.

In the area of the silylalkylphosphine intermediates of the present invention, there are several disclosures related to the present invention. British Patent No. 925,721 by H. Niebergall (assigned to Koppers Co., Inc.) broadly disclosed the addition of secondary phosphines to unsaturated silanes to provide silylalkyl phosphines. British Patent No. 1,179,242 by W. J. Owen and B. E. Cooper, assigned to Midland Silicones, Ltd., disclosed the preparation of similar compounds via displacement reactions of chlorophosphines and silylalkyl Grignard compounds or sodium phosphines and silylalkyl halides. The preparation of related compounds, i.e., alkoxysilylalkylphosphines was described via an alternative addition method reacting alkoxysilances and unsaturated phosphines, by F. Fekete in U.S. Patent No. 3,067,227, assigned to Union Carbide Corp. Silylalkylphosphine intermediates useful in the preparation of the complexes of the present invention were disclosed by J. K. Jacques and W. J. Owen in British Patent No. 1,182,763 assigned to Albright and Wilson (MFG) Ltd., by B. E. Cooper and W. J. Owen in a journal article on oxidation potentials [see J. Organometal. Chem., 29, 33—40 (1971)].

In the area of insoluble, anchored phosphinetransition metal complex catalysts reactive silyl substituted alkyl diphenyl phosphines were utilized as intermediates for anchoring. For reference, see U.S. Patent No. 3,726,809 by K. G. Allum, S. McKenzie and R. C. Pitkethly and U.S. Patent No. 3,907,852 by A. A. Oswald and L. L. Murrell. Such phosphine anchoring agents had at least one reactive substituent on the silicon. As such, they reacted with the surface hydroxyl group of silica via siloxane formation.

In contrast to the prior art, it was found in the present invention that tris-(alkyl diaryl phosphine) rhodium carbonyl hydride complexes are attractive selective hydroformylation catalysts in the absence of TPP-rhodium, dependent on several unexpected conditions. Compared to the widely studied triphenyl phosphine rhodium complexes, the optimum catalysis temperature of the present complexes is higher. Higher hydroformylation temperatures using the present catalysts are possible because catalyst stability and selectivity are better maintained.

One of the key unexpected factors in process of the present invention is that the present catalysts can be employed in a large excess without a drastic loss of catalyst activity. The other factor, also important for high selectivity, is the high ratio $H_2$ to CO. Unexpectedly, the excess of hydrogen does not result in the reduction of the aldehyde hydroformylation products to the corresponding alcohols. Coupled with the high $H_2$/CO ratios, it is essential in the present process to employ relatively low pressures, effectively limiting the CO partial pressure. Finally, the continuous process of the present invention is distinguished by relatively low olefin conversions. These are important for both catalyst stability and selectivity.

Due to the above characteristics, the present alkyl diaryl phosphine complex catalysts are uniquely suited for an operation wherein the aldehyde product is separated from the catalyst by distillation. Such a specifically advantageous operation is carried out in a continuous fashion wherein the olefin and synthesis gas feed are continuously introduced into the reactor comprising the catalyst solution and a mixture of the aldehyde product and the feed is continuously withdrawn in the gas phase.

The preferred selective process of the present invention, particularly the combination of the above features, is unique. It is not only unexpected in view of the prior art but was described as a process which should be inoperative due to the type of phosphine ligands employed.

When compared to the tris-(triphenyl phosphine) rhodium carbonyl hydride (TPP-rhodium) plus triphenyl phosphine based commercial, continuous process, the present process exhibits surprising advantages. The alkyl diaryl phosphines of the present process do not undergo P-C bond scission. The only catalyst by-products are the corresponding phosphine oxides. The latter are not inhibitors. The secondary by-products derived from the aldehyde products such as aldehyde trimers do not seriously inhibit the present catalytic system either. The present catalysts stand out with regard to long term activity maintenance in a continuous process. In contrast to the known process, no introduction of oxygen and/or chelating compounds or use of hydroxylic solvent is required for activity maintenance. As a consequence of higher catalyst stability, the present process can be operated at higher temperatures. This, in turn, can lead to an improved product to feed ratio in the distillate of the continuous product flash-off process. Also, it extends applicability to higher olefins and olefin derivatives. In addition, it provides unexpected advantages when employed for combined hydroformylation-aldolization-hydrogenation processes.

The applicability of the present phosphine ligands unexpectedly but understandably depends on their steric requirements, too. Substituents on the alkyl moiety close to the phosphorus were found to inhibit

phosphine complexation with rhodium compounds for the first time. In contrast, substituents outside the immediate proximity of phosphorus resulted in improved complex catalysts. Such substituted phosphines could be surprisingly advantageously produced via the addition of diaryl phosphines to vinyl compounds having activated double bonds.

The alkyl diaryl phosphines of the present invention were found to complex with rhodium more strongly than triaryl phosphines. This finding led to a novel method of producing the present catalysts via ligand displacement, e.g., by the reaction of alkyl diaryl phosphines with tris-(triphenyl phosphine) rhodium carbonyl hydride. Accordingly to another novel method, the present complexes are produced from acetylacetonato dicarbonyl rhodium either prior to use or *in situ* under the reaction conditions.

The present invention concerns novel bis- and tris-(alkyl diaryl phosphine) rhodium carbonyl complexes and a novel hydroformylation process using said complexes.

The present complexes usually contain 2 or 3, preferably 3, coordinated alkyl diaryl phosphine moieties per rhodium, and contain one carbon monoxide ligand per rhodium, and one hydride ligand per rhodium.

The broad class of bis- or tris- alkyl diaryl phosphine complexes is of the general formula

$$[(Ar_2PQ)_bE^yR_{y-b}]_g \cdot [Rh(CO)H]_s \qquad I$$

wherein Ar is an aryl group containing from 6 to 10 carbon atoms;

Q is an alkylene radical or an alkylene radical, the carbon chain of which is interrupted with at least one ether oxygen or phenylene group, wherein the alkylene radical contains from 2 to 30 carbon atoms;

E represents 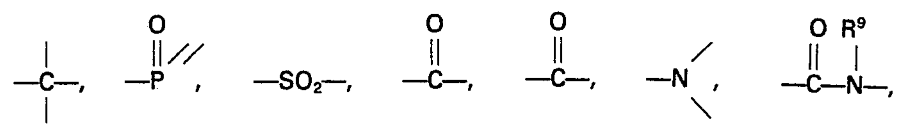

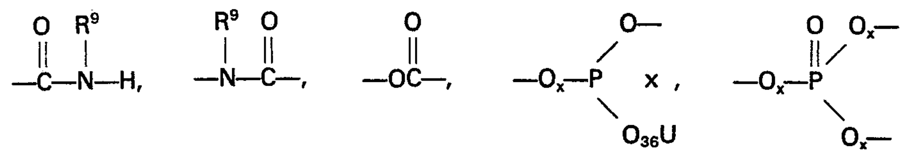

—O— or —S—, wherein $R^9$ is H, an alkyl group containing 1 to 30 carbon atoms or an aryl group containing from 6 to 10 carbon atoms, and wherein x is 0 or 1 with the proviso that at least one x is 1;

y represents the number of bonds available from the group E for attachment to the groups Q and R;

R independently represents an alkyl group containing from 1 to 30 carbon atoms or an aryl group containing from 6 to 10 carbon atoms with the proviso that when

E is 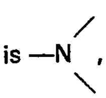, y=3 and b=1, R is $R_2$, the divalent radical $R^4$,

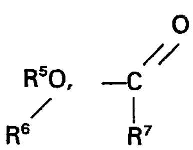 or 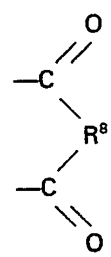 which

together with the N atom forms a heterocyclic ring, wherein $R^4$, $R^5$, $R^6$, $R^7$ and $R^8$ are hydrocarbyl radicals such that said heterocyclic ring contains not more than 15 atoms;

b is an integer of from 1 to 4, provided that y-b is not less than zero.

g is 1 to 6 with the proviso that

g times b is 1 to 6; and

s is 1 to 3.

Another class of compounds of the present invention include those of the formula

$$[(Ar_2PQ)_bSiR_{4-b}]_g(MX_n)_s$$

wherein Ar, Q, b, R, g, n and s are as defined above,

X is an anion or organic ligand, excluding halogen, satisfying the coordination sites of the metal. If X is

a metal it is preferably a non-coordinating anion and preferably borate, aluminate, perchlorate, sulphonate, nitrage, fluorophosphate or fluorosilicate such as $pH_4B^-$, $F_4B^-$, $C1O_4^-$, $Ph_3SO_3^-$, $NO_3^-$, $CF_3SO_3^-$, $F_6P^-$, or $F_6Si^{2-}$ and M is Co, Rh, Ir, Ru, Fe or Os. X is not preferably (Co)H.

The preferred substituents of the aromatic groups are $C_1$ to $C_{30}$, preferably $C_1$ to $C_{12}$ alkyl, alkoxy, acyl, acyloxy, acrylamido, carbamido, carbohydrocarbyloxy, halogen, phenoxy, hydroxy, carboxy. These substituents are preferably bound to a phenyl group. Mono- and di-substituted phenyl groups are preferred.

Examples of the aromatic groups are phenyl, fluorophenyl, difluorophenyl, tolyl, xylyl, benzoyloxyphenyl, carboethoxyphenyl, acetylphenyl, ethoxyphenyl, phenoxyphenyl, biphenyl, naphthyl, hydroxyphenyl, carboxyphenyl, trifluoromethylphenyl, tetrahydronaphthyl, furyl, pyrryl, methoxyethylphenyl, acetamidophenyl, dimethylcarbamylphenyl.

The alkyl groups are primary and secondary alkyl groups, preferably primary alkyl groups. Next to the preferred primary α-carbons of the alkyl groups, the β-carbons are primary and secondary, preferably also primary.

Exemplary alkyl groups are methyl, n-hexyl, docosyl, triacontyl, fluoropropyl, perfluoroethyl-ethyl, isopropyl, primary isobutyl, cyclopentyl, t-butylethyl, cyclohexylethyl, phenylethyl, trimethylsilylethyl, hydroxy, methoxyethoxyethyl, acetylethyl, pyrrolidinonylethyl, tributylphosphonium substituted ethyl, tris-hydroxy substituted t-butylethyl, triphenylmethylethyl, hydroxypropyl, carbomethoxyethyl, phenoxyethyl, benzamidoethyl, benzoyloxyethyl, pyrrylethyl, furylethyl, thienylethyl. The nonhydrocarbyl, i.e., heteroorganic R′ groups will be further defined.

The alkyl groups as defined by R′ are mono- or polyvalent alkyl groups, their valence ranging from 1 to 4.

Particularly preferred complexes within this class are complexes of the formulae

$$[(Ar_2P(CH_2)_m)_bSiR_{4-b}]_g[Rh(CO)H]_s,$$

$$[(Ph_2PQ)_bSiR_{4-b}]_g[Rh(CO)H]_s,$$

$$[(Ph_2P(CH_2)_m)_bSiR_{4-b}]_g[Rh(CO)H]_s,$$

$$[(Ar_2P(CH_2)_m)_2Si(CH_3)_2]_3[Rh(CO)H]$$

$$(Ph_2PQSiR_3)_3Rh(CO)H,$$

$$(Ph_2P(CH_2)_mSiR_3)_3Rh(CO)H,$$

$$(Ar_2PQSiR_3)_2Rh^+(CO)_3G^-,$$

$$[Ar_2P(CH_2)_mSiR_3]_2Rh^+(CO)_3G^-, \text{ and}$$

$$[Ar_2P(CH_2)_mSiR_3]_2Rh^+(CO)_3(BPh_4^-)$$

wherein the symbols are as defined above and $G^-$ is $X^-$. Particularly preferred compounds are

$$[Ph_2P(CH_2)_2Si(CH_3)_3]Rh(CO)H$$

$$[Ph_2P(CH_2)_3Si(CH_3)_3]Rh(CO)H,$$

$$[Ph_2P(CH_2)_2Si(C_3H_7)_3]Rh(CO)H,$$

$$[Ph_2P(CH_2)_2SiPh_3]Rh(CO)H, \text{ and}$$

$$[(Ph_2PCH_2CH_2)_2Si(CH_3)_2]_3[Rh(CO)H].$$

Still another preferred class of novel compounds within the scope of the present invention include those in which E represents a tertiary carbon group, i.e., catalysts of the formulae

$$[(Ar_2PQ)_bCR_{4-b}]_g \cdot [Rh(CO)H]_s$$

and

$$(Ar_2PQCR_3)_3Rh(CO)H$$

wherein Ar, Q, R, X, b, g, n and s are as defined above. A particularly preferred class of such compounds include those of the formula

$$(Ph_2P-(CH_2)_m-CR_3)_3Rh(CO)H$$

wherein m is an integer of from 2 to 30 and R is as defined above. Examples of such catalysts include

$$(Ph_2PCH_2C(CH_3)_3)_3Rh(CO)H,$$

$$[Ph_2PCH_2CH_2C(CH_3)_3]_3Rh(CO)H$$

and

$$(Ph_2PCH_2CH_2CH_2C(CH_3)_3)_3Rh(CO)H.$$

A further preferred class of compounds within the scope of the present invention include keto substituted compounds of the formulae

$$[(Ar_2PQ)_b\overset{O}{\overset{\|}{C}}R_{2-b}]_g \cdot [Rh(CO)H]_s$$

$$(Ar_2PQ\overset{O}{\overset{\|}{C}}R)_3Rh(CO)H$$

and

$$[(Ar_2PCH_2)_2\overset{O}{\overset{\|}{C}}R]_3[Rh(CO)H]_2$$

wherein Ar, Q, R, X, b, g, n and s are as defined above. Of such compounds particularly preferred are acyl compounds of the formula

$$[Ph_2P-(CH_2)_m-\overset{O}{\overset{\|}{C}}R]_3Rh(CO)H$$

wherein m is an integer of from 1 to 30, especially from 2 to 14, and R is as defined above. Examples of such catalysts include

$$(Ph_2PCH_2CH_2\overset{O}{\overset{\|}{C}}CH_3)_3Rh(CO)H,$$

$$(Ph_2PCH_2CH_2\overset{O}{\overset{\|}{C}}C_2H_5)_3Rh(CO)H$$

and

$$[Ph_2PCH_2CH_2\overset{O}{\overset{\|}{C}}Ph]_3Rh(CO)H.$$

Catalysts having carbohydrocarbyloxy substituted phosphines are of the formulae

$$\{[Ar_2P(CH_2)_m\overset{O}{\overset{\|}{C}}OCH_2]_2\}_3Rh(CO)H$$

$$(Ar_2PQ\overset{O}{\overset{\|}{C}}OR)_3Rh(CO)H$$

$$[Ar_2P(CH_2)_m\overset{O}{\overset{||}{C}}O_2R]_3Rh(CO)H$$

$$[Ph_2PCH_2CH_2\overset{O}{\overset{||}{C}}OCH_2]_3Rh(CO)H$$

and

$$[Ph_2PCH_2CH_2CO_2R]_3Rh(CO)H,$$

wherein Ar, Ph, Q, R, b, g, m, n and s are as defined above, represent another preferred class of compounds in accordance with the present invention. Particularly preferred are compounds of the formula

$$(Ph_2P\text{-}(CH_2)_m\text{-}\overset{O}{\overset{||}{C}}OR)_3Rh(CO)H$$

wherein m is an integer of from 2 to 22, especially from 4 to 14, and R is as defined above. Examples of compounds within this class include

$$(Ph_2PCH_2CH_2\overset{O}{\overset{||}{C}}OCH_3)_3RhH(CO)$$

and

$$[(Ph_2PCH_2CH_2CO_2CH_2)_2]_3[Rh(CO)H]_2.$$

The corresponding acyloxy complexes are also included within the novel carboxylate substituted complexes of the present invention. These complexes are of the formula

$$[(Ar_2PQ)_bO\overset{O}{\overset{||}{C}}R_{2-b}]_g[Rh(CO)H]_s$$

$$[Ar_2PQO\overset{O}{\overset{||}{C}}R]_3Rh(CO)H$$

$$[Ar_2P(CH_2)_mO\overset{O}{\overset{||}{C}}R]_3Rh(CO)H$$

$$[Ar_2PCH_2CH_2O\overset{O}{\overset{||}{C}}Ph]_3Rh(CO)H$$

and

$$[(Ar_2PCH_2CH_2O\overset{O}{\overset{||}{C}}CH_2)_2]_3[Rh(CO)H]_2$$

wherein Ar, Q, R, X, b, g, n, m and s are as defined above. Exemplary of suitable compounds within this class is

$$[Ph_2PCH_2CH_2O\overset{O}{\overset{||}{C}}H_2]_3Rh(CO)H$$

wherein Ph is phenyl.

Non-chelating trivalent nitrogen substituted complexes of the formula

$$[(Ar_2PQ)_bNR_{3-b}]_g[Rh(CO)H]_s$$

wherein Ar, Q, b, g, s, n, R and X are as defined above, represent another class of preferred compounds of the invention. These complexes include non-chelating open chain amino substituted alkyl diaryl phosphine complexes and nitrogen-containing heterocyclic ring substituted alkyl diaryl phosphine complexes. The latter include cyclic amides and imides. Preferred complexes within this class include amino substituted complexes of the formulae:

$$[(Ar_2PQ)_bNR_{3-b}]_g[Rh(CO)H]_s$$

$$[Ar_2PQNR^4]_3Rh(CO)H$$

$$[Ar_2PQNR_2]_3Rh(CO)H$$

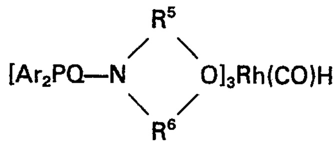

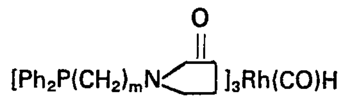

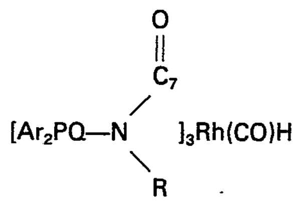

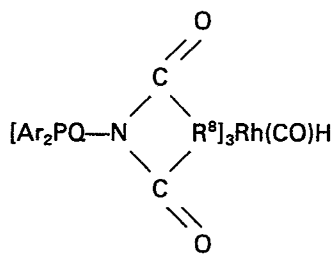

$$[Ph_2P(CH_2)_mN(C_pH_{2p+1})_2]_3Rh(CO)H$$

and

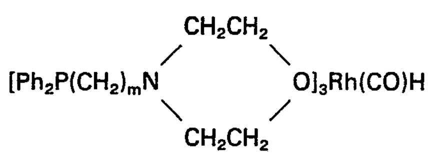

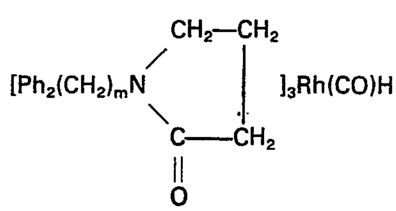

wherein Ar, Q, b, R, g, s, $R^4$, $R^5$, $R^6$, $R^7$, $R^8$ and pH are as defined above and p is an integer of from 1 to 12.

Examples of such compounds include

$$[Ph_2PCH_2CH_2N(C_2H_5)_2]_3Rh(CO)H,$$

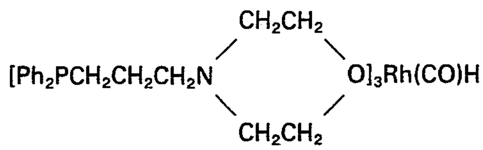

and

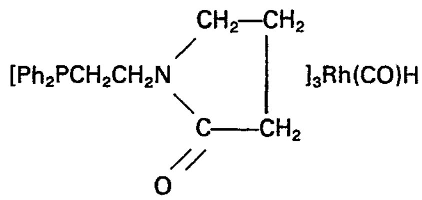

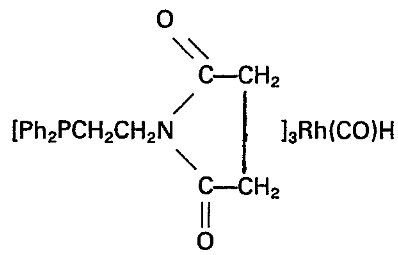

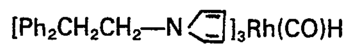

Another class of nitrogen-containing complexes of the invention are amide substituted open chain alkyl diaryl phosphine complexes of the formula

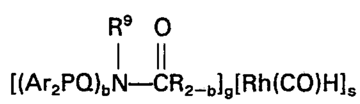

wherein Ar, Q, b, g, s, n, $R^9$, R and X are as defined above. Preferably, $R^9$ is H, an alkyl group containing 1 to 6 carbon atoms or phenyl. Preferred complexes within this class are of the formulae:

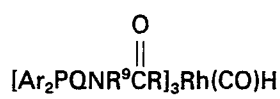

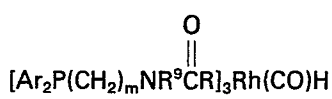

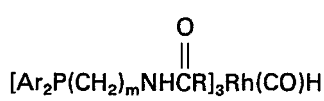

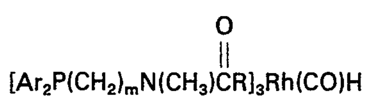

and

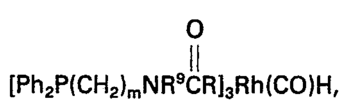

wherein Ar, Q, $R^9$, R, m and Ph are as defined above.

Examples of such compounds are

$$[Ph_2PCH_2CH_2CONH_2]_3Rh(CO)H$$

and

$$[Ph_2PCH_2CH_2CON(CH_3)_2]_3Rh(CO)H$$

The novel complexes of the invention also include carbamic acid derivatives of alkyl diaryl phosphine complexes of the formulae:

$$[(Ar_2PQ)_b\overset{\overset{O}{\|}}{C}—NR^9_{2-b}]_g[Rh(CO)H]_s$$

wherein Ar, Q, $R^9$, b, g, n and s are as defined above. Preferred complexes within this class include

$$[Ar_2PQ\overset{\overset{O}{\|}}{C}NR^9_2]_3Rh(CO)H$$

$$[Ar_2P(CH_2)_m\overset{\overset{O}{\|}}{C}NR^9_2]_3Rh(CO)H$$

$$[Ar_2PCH_2CH_2\overset{\overset{O}{\|}}{C}NR^9_2]_3Rh(CO)H$$

$$[Ar_2PCH_2CH_2\overset{\overset{O}{\|}}{C}NHR^9]_3Rh(CO)H$$

and

$$[Ph_2PCH_2CH_2\overset{\overset{O}{\|}}{C}NR^9_2]_3Rh(CO)H,$$

wherein the symbols have the above defined meanings. An example of such a compound is

$$[Ph_2PCH_2CH_2\overset{\overset{O}{\|}}{C}N(CH_3)_2]Rh(CO)H$$

The novel compounds of the present invention also include trivalent phosphorus derivatives of the formula

$$[Ar_2PQ(O)_xP\begin{matrix}\diagup (O)_xR\\ \diagdown (O)_xR\end{matrix}]_3Rh(CO)H$$

wherein Ar, Q, R, X, b, x, g, n and s are as defined above. Examples of such compounds include

$$[Ar_2PQOP(OR)_2]_3Rh(CO)H$$

$$[Ar_2P(CH_2)_mOP(OR)_2]_3Rh(CO)H$$

and

$$\{[Ar_2P(CH_2)_mO]_3P\}_3Rh(CO)H$$

wherein Ar, Q, R and m are as defined above. A preferred complex is

$[(Ph_2PCH_2CH_2CH_2O)_3P]_3Rh(CO)H.$

Still another preferred class of compounds in accordance with the present invention include higher valent phosphorus derivatives of the formula

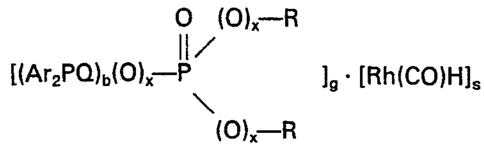

Preferred complexes within this class are

$$(Ar_2PQ\overset{O}{\overset{\|}{P}}R_2)_3RhH(CO),$$

$$[Ar_2PQ\overset{O}{\overset{\|}{P}}(OR)_2]_3Rh(CO)H$$

$$[Ar_2P(CH_2)_m\overset{O}{\overset{\|}{P}}R_2]_3Rh(CO)H$$

$$[(Ar_2P(CH_2)_m)_3\overset{O}{\overset{\|}{P}}]_3Rh(CO)H$$

$$[Ar_2P(CH_2)_m\overset{O}{\overset{\|}{P}}Ar_2]_3Rh(CO)H$$

$$[Ph_2P(CH_2)_m\overset{O}{\overset{\|}{P}}Ph_2]_3Rh(CO)H$$

and

$$[(Ar_2P(CH_2)_mO)_3\overset{O}{\overset{\|}{P}}]_3Rh(CO)H$$

wherein Ar, Q, R, b, x, g, m, Ph and s are as defined above. A preferred compound is of the formula

$$[Ph_2P\text{-}(CH_2)_m\text{-}\overset{O}{\overset{\|}{P}}R_2]_3Rh(CO)H$$

wherein m is an integer of from 1 to 30, especially from 2 to 14, and R is defined as above. One example of this type catalyst is

$$(Ph_2PCH_2CH_2\overset{O}{\overset{\|}{P}}Ph_2)_3Rh(CO)H.$$

Still another group of novel compounds within the scope of the present invention are the sulfone derivatives of the formula

$$[(Ar_2PQ)_bSO_2R_{2-b}]_g[Rh(CO)H]_s$$

wherein Ar, Q, R, b, g, n, and s are as defined above. Preferred complexes within this class include complexes of the formulae

$$[Ar_2PQSO_2R]_3Rh(CO)H$$

$$[Ar_2P(CH_2)_mSO_2R]_3Rh(CO)H$$

$$([Ar_2P(CH_2)_m]_2SO_2)_3[Rh(CO)H]_2$$

and

$$[Ar_2PCH_2CH_2SO_2R]_3Rh(CO)H$$

wherein Ar, Q, R, X, b, g, n, m and s are as defined above. A specific example of a compound within this class is

$$[Ph_2CH_2CH_2SO_2C_2H_5]_3Rh(CO)H$$

Ether derivatives of alkyl diaryl phosphine complexes of the formula

$$[(Ar_2PQ)_bOR_{2-b}]_g[Rh(CO)H]_s$$

wherein Ar, Q, R, g, b, n, s, and X are as defined above, form another class of the novel complexes of the invention. Preferred complexes within this class include complexes of the formulae:

$$[Ar_2PQOR]_3Rh(CO)H$$

$$[Ar_2P(CH_2)_mOR]_3Rh(CO)H$$

$$[Ar_2P(CH_2CH_2O)_3CH_3]_3Rh(CO)H$$

and

$$([Ar_2P(CH_2)_m]_2O)_3[Rh(CO)H]_2$$

wherein Ar, Q, R and m are as defined above. Exemplary of suitable compounds within this class is

$$[Ph_2PCH_2CH_2OPh]_3Rh(CO)H.$$

Also included within the scope of the novel complexes in the invention are hydroxy derivatives of the formulae

$$[Ar_2PQOH]_g[Rh(CO)H]_s$$

$$(Ar_2PQOH)_3Rh(CO)H$$

$$[Ar_2P(CH_2)_mOH]_3Rh(CO)H$$

and

$$[Ar_2PCH_2CH(OH)CH_2OH]_3Rh(CO)H$$

wherein Ar, Q, n, m, g and s are as defined above. Exemplary of a suitable compound within this class is

$$[Ph_2PCH_2CH_2CH_2OH]_3Rh(CO)H$$

Yet another class of novel compounds within the present invention are thioether derivatives of the formulae

$$[(Ar_2PQ)_bSR_{2-b}]_gRh(CO)H$$

$$[Ar_2PQSR]_3Rh(CO)H$$

and

$$[Ar_2P(CH_2)_mSR]_3Rh(CO)H$$

wherein Ar, Q, R, X, b, g, n, m and s are as defined above. Exemplary of suitable compounds within this class is

$$[Ph_2PCH_2CH_2CH_2SPh]_3Rh(CO)H$$

The diaryl alkyl phosphine ligands employed in the present invention are prepared by any number of standard techniques. One preferred synthesis technique involves the addition of diaryl alkyl phosphines to suitable unsaturated compounds:

$$bAr_2PH+[CH_2{=}CH{-}D{+}_bE^yR_{y-b}\rightarrow[Ar_2PCH_2CH_2D{+}_bE^yR_{y-b}$$

or

$$[Ar_2PQ]_bE^yR_{y-b}$$

wherein $CH_2{=}CHD{-}$ after the reaction represents the group Q as defined above. Thus, D can be a covalent bond or the remainder of the group Q other than $-CH_2-CH_2-$. Preferably $-D-$ represents $-(CH_2)_k-$ wherein k ranges from 0 to 28, especially from 0 to 6 and most preferably from 0 to 1. Such additions are preferably carried out via a radical chain mechanism in a free radical manner. Chemical and/or radiation initiators can be used. It has been found that the selectivity of such reactions can be improved by using an excess of phosphine reactant, preferably from 5 to 100% excess over the stoichiometric amount required of the phosphine. A suitable chemical initiator for this process is a labile azo compound such as azo-bis-i-butyronitrile. The amount of the initiator can vary depending upon the chain length of the reaction and preferably is in the range of from about 0.1 to about 3%. The reaction temperature of a chemically initiated addition depends upon the temperature of initiating radical generation. This initiation normally occurs in the range of from 0 to 50°C.

For radiation initiation of the above-described addition reaction, ultraviolet light or gamma irradiation can be employed. The radiation intensity and duration are highly dependent upon the chain length, i.e., the G value. The preferred temperature of radiation initiation is between about −90°C. and +90°C.

The above-described radical addition of diaryl phosphines to certain substituted vinylic compounds is unexpectedly selective and fast. Such substituted vinylic compounds include vinyl silanes, vinyl ketones, N-vinyl amides, acrylates, and sulfones, as illustrated by the following formulae:

$$Ar_2PH+(CH_2{=}CH)_bSiR_{4-b}\rightarrow(Ar_2PCH_2CH_2)_bSiR_{4-b}$$

$$Ar_2PH+CH_2{=}CHSiR_3\rightarrow Ar_2PCH_2CH_2SiR_3$$

$$Ar_2PH+CH_2{=}CHNR^9COR\rightarrow Ar_2PCH_2CH_2NR^9COR$$

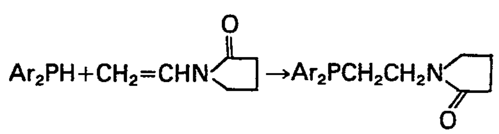

$$Ar_2PH+CH_2{=}CHCOR\rightarrow Ar_2PCH_2CH_2COR$$

$$Ar_2PH+CH_2{=}CHCO_2R\rightarrow Ar_2PCH_2CH_2CO_2R$$

$$Ar_2PH+CH_2{=}CHSO_2R\rightarrow Ar_2PCH_2CH_2SO_2R$$

The high reactivity of these substituted vinylic compounds is contrasted with the sluggish behavior of some other substituted olefins having analogous structures, e.g., t-butyl ethylene.

It has also been observed that additions of phosphines to vinylic compounds wherein k=0 occur with ease in the presence of radiation particularly ultraviolet light. The reactivity of such vinyl compounds is in marked contrast to the rather sluggish behavior of olefins having analogous structures. In addition to such vinyl compounds, allyl compounds wherein k=1 are another preferred class of reactant.

Similar anti-Markovnikov additions can be carried out via anionic mechanism with base catalysis to certain conjugated vinylic compounds such as acrylates. Such anionic addition can be performed with either added base catalysis, e.g., a quaternary base addition, or without any base added in addition to the phosphine base.

Another technique which may be employed in the preparation of the alkyl diaryl phosphine ligands involved in the present invention is the addition of suitable hydrogen-containing compounds to unsaturated phosphines, preferably vinyl diaryl phosphines:

$$bAr_2P{-}D{-}CH{=}CH_2+H_bE^yR_{y-b}\rightarrow[Ar_2P{-}D{-}CH_2{-}CH_2{+}_bE^yR_{y-b}$$

or

$$[Ar_2PQ]_bE^yR_{y-b}$$

wherein D is defined as above. Again, D preferably represents $-(CH_2)_k-$ wherein k is an integer of from 0 to 28. The preferred reactants are the vinylic and allylic materials, this time the phosphines. Thus, the heteroorganic substituted alkyl diaryl phosphines are derived by employing compounds such as secondary phosphine oxide, dihydrocarbyl hydrogen phosphites and thiols as the hydrogen-containing compound.

The above approach is exemplified by the free radical addition of secondary phosphine oxides, hydrogen phosphites and thiols:

$$Ar_2P(CH_2)_kCH{=}CH_2 + H\overset{O}{\overset{\|}{P}}R_2 \rightarrow Ar_2P(CH_2)_kCH_2CH_2\overset{O}{\overset{\|}{P}}R_2$$

$$Ar_2P(CH_2)_kCH{=}CH_2 + H\overset{O}{\overset{\|}{P}}(OR)_2 \rightarrow Ar_2P(CH_2)_kCH_2CH_2P(OR)_2$$

$$Ar_2P(CH_2)_kCH{=}CH_2 + HSR \rightarrow Ar_2P(CH_2)_kCH_2CH_2SR$$

wherein Ar, k and R are as defined above. It is surprising that similar additions could not be carried out using silanes as adding agents. In general, the reaction conditions for the successful radical additions were those previously described for the diaryl phosphine olefin additions.

Other methods for alkyl diaryl phosphine ligand preparation employ displacement reactions. One type of reaction starts with diaryl phosphides, particularly alkali metal phosphides, and suitable chloro-, bromo-, or iodo-alkyl compounds:

$$bAr_2PMe + [LQ]_bE^yR_{y-b} \rightarrow [Ar_2PQ]_bE^yR_{y-b} + bMeL$$

wherein Me is Na, K, Li and L is Cl, Br, I. Another technique starts with diaryl chloro or bromo phosphines and the corresponding Grignard derivatives of suitable alkyl compounds:

$$bAr_2PJ + [JMgQ]_bE^yR_{y-b} \rightarrow [Ar_2PQ]_bE^yR_{y-b} + bMgJ_2$$

wherein J is chlorine or bromine and the other symbols are as defined above.

For the preparation of the present complexes, standard methods of organometallic chemistry are discussed in a comprehensive text, "Advanced Inorganic Chemistry," by F. A. Cotton and G. Wilkinson (Interscience Publishers, New York, 1972) and exemplified in the series on "Inorganic Syntheses" particularly Volume XV, edited by G. W. Parshall and published by McGraw-Hill Book Co., New York, 1974, and in U.S. Patent No. 4,052,461 by H. B. Tinker and D. E. Morris, assigned to Monsanto Co.

For the preparation of the rhodium complexes, one of the specifically preferred direct methods of synthesis starts with rhodium chloride. This method can be employed, e.g., for the synthesis of tris-(alkyl diaryl phosphine) rhodium carbonyl hydride complexes according to the following general scheme:

$$RhCl_3 \cdot 3H_2O \xrightarrow[CH_2O\ aq.,\ KOH\ ethanolic]{Ar_2PQY\ (excess)} [Ar_2PQY]_3Rh(CO)H$$

Other preferred direct methods of complex preparation include the reaction of transition metal carbonyls or oxides, such as those of rhodium with suitable diaryl alkyl phosphine ligand and $CO/H_2$. Similarly, organic salts of transition metals such as acetates can be reacted with the diaryl alkyl phosphine ligand.

According to one preferred method, the rhodium reactant is a rhodium salt, preferably rhodium trichloride or its hydrate. This method preferably employs a base, most preferably KOH or sodium borohydride, and a reducing carbonylating agent such as formaldehyde, hydrogen and CO to produce the carbonyl hydride complex via the tris-(alkyl diaryl phosphine) rhodium chloride and its carbonyl derivative intermediate compounds.

The complexes of the invention can also be prepared via an indirect method by reaction of the corresponding complexes of a triaryl phosphine, preferably triphenyl phosphine, with the desired diarylalkyl phosphine ligand, preferably in excess, e.g.:

$$(Ph_3P)_3Rh(CO)H + 3Ar_2PQE^yR_{y-1} \rightarrow (Ar_2PQE^yR_{y-1})_3Rh(CO)H + 3Ph_3P$$

In general, the alkyl diaryl phosphine ligands are more basic than the corresponding triaryl phosphines. This basicity difference is a positive factor in the above ligand substitutions, providing completely or partially exchanged complexes, e.g.:

$$(Ph_2PR)(Ph_3P)_2Rh(CO)H \xrightarrow{Ph_2PR} (Ph_2PR)_2(Ph_3P)Rh(CO)H$$

$$\downarrow Ph_2PR$$

$$(PhPR)_3Rh(CO)H$$

The formation of the tris(alkyl diaryl phosphine) rhodium carbonyl hydride complexes via ligand exchange can be followed by $^{31}P$ nuclear magnetic resonance. $^{31}P$ nmr can be also used for the identification and the quantitative determination of the starting alkyl diaryl phosphine reactants. As the ion exchange proceeds in an appropriate inert solvent, preferably aromatic hydrocarbon, $^{31}P$ nmr shows the number and amounts of the different phosphine species. When an excess of the theoretically required alkyl diaryl phosphine reactant, preferably more than 100% excess, is used, the formation of the tris(alkyl diaryl phosphine) complex is essentially quantitative. The reaction temperature is between 10 and 100°C., usually ambient temperature.

Ligand exchange methods can be used for the preparation of the present complexes *in situ*, e.g., under hydroformylation conditions. For this purpose, the various rhodium carbonyls, and appropriate organic salts of rhodium carbonyl are particularly preferred. For example, dicarbonyl acetylacetonato ($AcAc^-$) dicarbonyl rhodium can be reacted with hydrogen and an excess amount of alkyl diaryl phosphine:

$$Rh^+(CO)_2(AcAc^-)+3Ar_2PR+H_2 \rightarrow (Ar_2PR)_3Rh(CO)H+CO+(CH_3CO)_2CH_2$$

The synthesis and the physicochemical constants of most of the known phosphines is given in Volume 1 of a series of monograph, entitled "Organic Phosphorus Compounds," edited by G. M. Kosolapoff and L. Maier, published by J. Wiley and Sons, Inc., New York, N.Y., in 1972. Particularly, Chapter 1 on "Primary, Secondary and Tertiary Phosphines," by L. Maier, is relevant. Specifically, pages 33 to 105 and 135 to 224 provide the information on the certain tertiary phosphines used in the present invention. Chapter 3A, particularly pages 433 to 493, on "Phosphine Complexes with Metals" by G. Booth of the same volume also provides general information regarding phosphine rhodium complexes.

The novel complexes of the present invention have been found to be particularly useful in carbonylation reactions, particularly hydroformylation reactions, which involve the reaction of unsaturated organic compounds with CO, or CO and hydrogen mixtures. Carbonylation reactions are generally reactions of unsaturated organic compounds with carbon monoxide plus preferably a third reactant. Carbonylations are described in detail in the earlier referenced Falbe monograph. Main types of carbonylations catalyzed by the present complexes are the Roelen reaction (hydroformylation) of olefins with CO and $H_2$ and subsequent aldolization reactions: the Reppe reaction (metal carbonyl catalyzed carbonylation) mainly of olefins, acetylenes, alcohols and activated chlorides with CO alone or with CO plus either alcohol or amine or water; and ring closure reactions of functional unsaturated compounds such as unsaturated amides with CO. The unsaturated organic reactants are preferably olefinically unsaturated compounds, more preferably olefinic hydrocarbons.

The most preferred carbonylation is the improved, selective hydroformylation process of the present invention, however, the novel complexes of the invention can be employed as catalysts in other prior art methods to obtain good results.

The most preferred carbonylation is an improved, selective hydroformylation comprising reacting an olefin with a mixture of carbon monoxide and hydrogen in the presence of an alkyl diaryl phosphine halogen free rhodium complex as a catalyst to produce mainly an aldehyde, preferably via carbonylation at the less substituted vinylic carbon. Halogen free means that there is no reactive halogen, particularly chlorine, bonded to rhodium.

An improved method for hydroformylation was discovered comprising reacting an olefin with CO and $H_2$ in the presence of a tris- and bis-(alkyl diaryl phosphine) rhodium carbonyl complex catalyst free from halogen on the rhodium and excess free tertiary phosphine ligand wherein said improvement is effected by an appropriately high ratio of both $H_2$/CO and ligand/Rh to produce a selective catalyst system of improved thermal stability and long term operational stability which leads to a ratio above four of n- and i-aldehyde primary products said products being the major primary products when the method employs a 1-n-olefin as starting reactant.

The preferred complex catalysts are nonchelating tris-(substituted alkyl diaryl phosphine) rhodium carbonyl hydride complex compounds of the formula:

$$[Ar_2P(CH_2)_mER_y''']_3Rh(CO)H$$

wherein the meaning of the symbols is as previously defined, except ER''' is a nonchelating heteroorganic radical, preferably being selected from silane silicone; ether, ester, keto and hydroxy oxygen; phosphine, phosphonium and phosphorus ester phosphorus; amine, amido, heterocyclic and ammonium nitrogen; sulfide and sulfone sulfur.

In hydroformylation reactions employing the novel complexes of the invention, organic non-hydrocarbon solvents, preferably of weak, nonsubstituting ligand character, are advantageously used. Preferred solvents of ligand character are triaryl phosphines, such as triphenyl phosphine, triaryl stibines, triaryl arsines. Other preferred organic solvents are ketones such as acetophenone, diphenyl ketone, polyethylene glycol and organic silicone compounds such as diphenyl dipropyl silane. More preferred ligand solvents are triaryl phosphines. Of course, the most preferred solvent is one used in the process of the present invention as described above containing an excess of an alkyl phosphine ligand, preferably the

same alkyl diaryl ligand as complexed with the $[Rh(CO)H]_s$ group. This last reaction system has been found to provide particularly advantageous results as explained in the following.

As far as other prior art carbonylation processes employing the present novel complexes are concerned such processes can be performed advantageously under the usual conditions such as those described in the earlier referenced Falbe monograph. Generally, the olefins described above can be employed in processes using the novel complexes of the invention. However, prior art processes employing the novel catalysts will normally have to be performed at higher temperature because although the novel complexes have greater stability and selectivity than triaryl phosphine complexes previously employed, the novel complexes generally provide relatively lower reaction rates. Typical total pressures, rhodium concentrations, ligand concentration and $H_2$ to CO partial pressure ratios for such prior art processes are

Total pressure: 30 to 30000 psi
Rh concentration: 10 to 10000 ppm
Ligand concentration: 100 to 200,000 ppm

Moreover, in contrast to the process of the present invention, these prior art processes can employ an excess of any tertiary phosphine, including triphenyl phosphine. However, if the excess ligands include phosphines having structures other than that of the complexed diaryl alkyl phosphine, such ligand should not displace more than one of the three ligands of the novel tris-(diaryl alkyl phosphine) rhodium complex of the invention. The other complexing groups present in substituted alkyl diaryl phosphines should not effect such a multiple substitution either.

The novel complexes of the invention can also be employed in combined processes such as a combined hydroformylation/aldolization. For example, a process employing the normal prior art hydroformylation condition discussed above along with an aldol condensation catalyst and a novel complex of the invention would be one such process.

In the drawings:

Figure 1 shows the key steps in the mechanism of phosphine-rhodium complex catalyzed hydroformylation of olefins.

Figure 2 illustrates a comparative $^{31}P$ NMR spectra of certain rhodium complexes.

Figure 3 illustrates a $^{31}P$ NMR study of certain ligand exchange rates as a function of temperature.

Figure 4 is a schematic representation of an autoclave for hydroformylation.

Figure 5A is a graphical comparison of certain catalyst stabilities at high temperature hydroformylation of butene-1.

Figure 5B is a graphical comparison of certain catalyst stabilities at low ligand/Rh ratio in Hydroformylation of butene-1.

Figure 6 is a graphical representation of butene hydroformylation rate versus temperature correlations in the presence of SEP and TPP based rhodium-phosphine complex catalysts.

Figure 7 is a graphical representation of 1-butene hydroformylation temperature versus n-valeraldehyde (n) to total valeraldehyde (n+i) ratio correlations in the presence of SEP and TPP based rhodium-phosphine complex catalysts.

Figure 8 is a graphical representation of 1-butene hydroformylation temperature versus butane by-product formation correlations in the presence of SEP and TPP based complex catalysts.

Figure 9 is a graphical representation of 1-butene hydroformylation temperature versus 2-butenes by-product formation correlations in the presence of SEP and TPP based complex catalysts.

Figure 10 is a graphical representation of the effect of SEP ligand to rhodium ratio on selectivity of butene hydroformulation at 145 and 170°C.

Figure 11 is a graphical representation of the effect of carbon monoxide pressure on ratio of n-valeraldehyde (n) to total valeraldehyde (n+i) product of 1-butene hydroformylation.

Figure 12 is a graphical representation of aldehyde production rate during continuous butene hydroformylation.

## Examples

### Preparation of alkyl diaryl phosphine ligands

With the exception of the available, simple alkyl diphenyl phosphine and alkylene bis-diphenyl phosphine laboratory chemicals, the ligand components of the present rhodium complexes were prepared during the present work.

The generally employed method for ligand preparation was the free radical chain addition of diphenyl phosphine to a vinylic compound in an anti-Markovnikov manner.

$$Ph_2PH+CH_2{=}CHR \rightarrow Ph_2PCH_2CH_2R$$

As a rule, such additions were initiated by broad spectrum ultraviolet light at 15°C. The rate of addition depended strongly on the type of the olefinic compound employed. In general, compounds having vinylic

substitution were highly reactive, while allylic derivatives were sluggish to react. The reaction times were accordingly varied. The selectivity of the additions could be improved by using more than the equivalent amount, generally 10% excess, of the phosphine adding agent. In the case of vinylic derivatives, this reduced the oligomerization of the unsaturated component. With allylic reactants, the phosphine excess suppressed the allylic reversal reactions of the radical intermediate.

If either the olefinic reactant or adduct product was immiscible with or insoluble in the diphenyl phosphine adding agent at 15°C, either the temperature was raised or a solvent was added or both were done to produce and maintain a homogeneous liquid reaction mixture. During the reaction, the conversion of reactants to products (and by-products) was followed by gas liquid chromatography (glc) and/or proton magnetic resonance spectroscopy (pmr). Usually the glc peak intensities were used to make quantitative estimates of the compositions. For identification of the product structures nmr was mainly used.

When the desired conversion was reached, the reaction mixture was usually fractionally distilled in high vacuo to obtain the pure adduct product. Most of the pure adducts were clear, colorless, mobile liquids at room temperature. In case of high melting products, recrystallization of the crude product was used as an alternate means of purification.

The expected structures of the isolated products were confirmed by pmr. Elemental analyses were also performed to check the product compositions.

The pure phosphines were studied to determine their basicity, directly by potentiometric titration and indirectly by $^{31}P$ nmr. The results of direct basicity determination will be given below, together with the other analytical characteristics of the free phosphine ligands. The $^{31}P$ nmr chemical shift values for the free ligands will be listed as comparative values when discussing the $^{31}P$ nmr of their rhodium complexes.

The phosphine basicity determinations via potentiometric titrations were performed according to the method of C. A. Streuli. For reference see Analytical Chemistry, Vol. 31, pages 1652 to 1654 in 1959 and Vol. 32, pages 985 to 987 in 1960. Half neutralization potentials (HNP's) of the phosphines were determined using perchloric acid as a titrant and pure nitromethane, free from weakly basic impurities, as a solvent. The values obtained were subtracted from the HNP of a stronger organic base, diphenyl guanidine, which served as a daily standard reference. The resulting ΔHNP values of the phosphines are indirectly related to their basicity. In case of phosphines which were also studied by Streuli, somewhat different HNP values were obtained in the present work. Since ion exchange resin purified nitromethane was used in the present work, the reported values should be more correct than Streulis'.

In the following, the preparation of different types of alkyl diaryl phosphine ligands will be described in the order of their subsequent use for forming rhodium complex catalysts. At first the trihydrocarbylsilyl substituted alkyl diphenyl phosphines will be discussed. They will be followed by simple alkyl diphenyl phosphines and alkylene bis-phosphines. Finally, the preparation of alkyl diphenyl phosphines having different types of substitution will be described. The successful use of a variety of ligands then exemplifies the broad scope of the invention.

Trihydrocarbylsilylethyl diphenyl phosphines
(Examples 1—6)

Six trihydrocarbylsilylethyl diphenyl silanes were prepared by adding diphenyl phosphine to the corresponding vinylic or allylic silane. The preparation, physical properties and analytical composition of the compound is summarized in Examples 1—6 below. The table also shows the basicity characteristics of the products as characterized by their ΔHNP values. It is noted that all the trihydrocarbylsilyalkyl diphenyl phosphines are much stronger bases than triphenyl phosphine ($Ph_3P$:ΔHNP=510).

Accounts of the individual experiments are given in the following.

Example 1
Preparation of trimethylsilylethyl diphenyl phosphine

$$Ph_2PH+CH_2=CHSi(CH_3)_3 \rightarrow Ph_2PCH_2/CH_2Si(CH_3)_3$$

A magnetically stirred mixture of 46.5 g (0.25 mole) diphenyl phosphine and 25 g (0.25 mole) of vinyl trimethyl silane in a closed cylindrical quartz tube was irradiated from about 3 cm distance with two 75 Watt Hanau tube immersion lamps, with a wide spectrum of ultraviolet irradiation in a 15°C. water bath for 26 hours. A proton magnetic resonance spectrum of a sample of the resulting mixture exhibited no significant peaks in the vinyl region indicating a substantially complete addition.

The reaction mixture was distilled in vacuo to obtain 61 g (81%) of the desired trimethylsilylethyl diphenyl phosphine adduct, as a clear colorless liquid, having a boiling range of 109—110°C. at 0.1 mm.

Anal. Calcd. for $C_{17}H_{23}PS$: C, 71.29; H, 8.09; P, 10.81. Found: C, 71.98; H, 8.12; P, 10.59. The ΔHNP (relative half neutralization potential compared to that of diphenyl guanidine) was 385.

The selectivity to provide the desired adduct was increased when the diphenyl phosphine reactant was employed in a 10 mole % excess.

Example 2
Preparation of tripropylsilylethyl diphenyl phosphine

$$ClSi(C_3H_7)_3 \xrightarrow{CH_2{=}CHMgBr} CH_2{=}CHSi(C_3H_7)_3 \xrightarrow{Ph_2PH} h_2PCH_2CH_2Si(C_3H_7)_3$$

To prepare the vinyl tripropyl silane reactant, chloro- tri-n-propyl silane was reacted with vinyl magnesium bromide in refluxing tetrahydrofuran. After removing the THF solvent by distillation, the residual product was taken up in ether, was washed with ice water and then with 5% aqueous sodium hydrogen carbonate. The ether solution was then dried over anhydrous sodium sulfate and distilled to obtain vinyl tripropyl silane, bp. 75—77°C at 11 mm.

The vinyl tripropyl silane was then reacted with diphenyl phosphine with u.v. initiation for 86 hours in a manner described in Example 1. The conversion was about 95%. The mixture was fractionally distilled to yield approximately 63% of the theoretical yield of the product as a clear, colorless, mobile liquid. The product distilled at 155—156°C at 0.10 mm.

Anal. Calcd. for $C_{23}H_{35}PSi$: C, 74.54; H, 9.52; P, 8.36 Found: C, 74.35; H, 923; P, 8.37. The ΔHNP of this compound was 385.

Example 3
Preparation of Triphenylsilylethyl diphenyl phosphine

$$CH_2{=}CHSiCl_3 \xrightarrow{3BrMgPh} CH_2{=}CHSiPh_3 \xrightarrow{Ph_2PH} Ph_2PCH_2CH_2SiPh_3$$

To obtain the vinyl triphenyl silane intermediate, vinyl trichloro silane was reacted with phenyl magnesium bromide in an ether-THF solvent mixture. The resulting product was worked up in a manner described in the previous example. The product was a low melting solid which could be distilled in vacuo using a hot condenser. At room temperature, the distillate solidified to yield a white crystalline compound, mp 60—65°C. Pmr confirmed the expected vinyl triphenyl silane structure.

Anal. Calcd. for $C_{20}H_{18}Si$: C 83.86; H, 6.33. Found: C, 83.92; H, 6.34. The ΔHNP of this compound was 385.

The vinyl triphenyl silane was reacted with 10% excess of diphenyl phosphine. To maintain a homogeneous reaction mixture, a temperature of 80°C and cyclohexane solvent were employed. After the usual u.v. initiated addition, the reaction mixture was allowed to cool to room temperature. This resulted in the crystallization of the triphenylsilylethyl diphenyl phosphine adduct. To obtain it in a pure form, the adduct was filtered and recrystallized from a four to one mixture of cyclohexane and toluene. A white crystalline product having a melting point of 128—131°C was obtained.

Anal. Calcd. for $C_{32}H_{24}Si$: C, 81.32; H, 6.19; P, 6.55. Found: C, 80.97; H, 6.18; P, 6.71. The ΔHNP of this compound was 413.

Example 4
Preparation of bis-(diphenylphosphinoethyl) dimethyl silane

$$(CH_3)_2Si(CH{=}CH_2)_2 \xrightarrow{Ph_2PH} Ph_2PCH_2CH_2Si(CH_3)_2CH{=}CH_2$$

$$\downarrow Ph_2PH$$

$$Ph_2PCH_2CH_2Si(CH_3)_2CH_2CH_2PPh_2$$

A mixture of 9.0 g (0.8 mole) dimethyl divinyl silane and 32.7 g (0.176) diphenyl phosphine (10% excess over equivalent amounts) was reacted for 22 hours in the manner described in Example 1. The reaction mixture was fractionated in vacuo to obtain minor amounts of the clear, colorless, slightly viscous liquid monoadduct, and major amounts of the clear, colorless, highly viscous liquid diadduct, i.e. the desired bis-(diphenylphosphinoethyl) dimethyl silane. The distillation yield of desired product was 84%. The bis-diphenylphosphinoethyl) dimethyl silane product had a boiling point of 238—239°C at 0.20 mm.

Anal. Calcd. for $C_{30}H_{34}P_2Si$: C, 74.35; H, 7.07; P, 12.78. Found: C 73.65; H, 6.90; P, 12.59. The ΔHNP of this compound was 434.

Example 5
Preparation of trimethylsilylpropyl diphenyl phosphine

$$Ph_2PH + CH_2{=}CHCH_2Si(CH_3)_3 \rightarrow Ph_2PCH_2CH_2CH_2Si(CH_3)_3$$

A mixture of 22.8 g (0.2 mole) allyl trimethyl silane and 37.2 g (0.2 mole) diphenyl phosphine was reacted for 158 hours in the manner described in Example 1. A subsequent fractional distillation, yielded

the desired pure adduct as a clear, colorless liquid. The distillation yield was 50%. The product had a boiling point of 150°C at 0.10 mm.

Anal. Calcd. for $C_{18}H_{25}PSi$: C, 71.96; H; 8.38; P, 10.31. Found: C, 72.27; H, 8.29; P, 10.25. The ΔHNP for this product was 408.

Example 6
Preparation of trimethylsilylmethyl diphenyl phosphine

$$Ph_2PLi+ClCH_2Si(CH_3)_3 \rightarrow Ph_2PCH_2Si(CH_3)_3$$

The known but unavailable trimethylsilylmethyl diphenyl phosphine was derived via reacting chloromethyl trimethyl silane with lithium diphenyl phosphide in an ether-hexane mixture. After removing the lithium chloride by-product by filtration, the product was isolated as a clear, colorless liquid by fractional distillation in vacuo. The distillation yield was 86% and the product had a boiling point of 129—130°C at 0.2 mm.

Anal. Calcd. for $C_{16}H_{21}PSi$: C, 70.55; H. 7.77; P, 11.37. Found: C, 70.01; H, 7.64; P, 11.36. The ΔHNP for the product was found to be 404.

Alkyl diaryl phosphines (Examples 7—15)

For a study of the influence of the alkyl structure in alkyl diphenyl phosphine ligands, a number of commercially available compounds were obtained (Table 1, Examples Nos. 7 to 11). The known but unavailable neopentyl diphenyl phosphine and 3,3-dimethylbutyl diphenyl phosphine (Table 1, Example Nos. 12 and 13, respectively) were synthesized by reacting the corresponding alkyl chlorides with lithium diphenyl phosphide.

Table 2 lists the relative half neutralization potentials of the various alkyl diphenyl phosphines. It is noted that as a group they are much more basic than triphenyl phosphine. The branching of the alkyl group, particularly in the proximity of the phosphorus, further increases the basicity.

TABLE 1
Alkyl diphenyl phosphine ligands and their basicity

| Example No. | Structure | Indirect basicity ΔHNP |
|---|---|---|
| 7[a] | $Ph_2PCH_2CH_3$ | 363 |
| 8[b] | $Ph_2PCH_2CH_2CH_3$ | 424 |
| 9[a] | $Ph_2PCH_2CH_2CH_2CH_3$ | 404 |
| 10[b] | $Ph_2PCH_2CH_2CH_2CH_2CH_2CH_3$ | 392 |
| 11[b] | $PhPCHCH_2CH_3$<br>$CH_3$ | 355 |
| 12[a] | $Ph_2PC(CH_3)_3$ | 341 |
| 13[c] | $Ph_2PCH_2C(CH_3)_3$ | 378 |
| 14[c] | $Ph_2PCH_2CH_2C(CH_3)_3$ | 412 |
| 15[a] | $Ph_2P$— 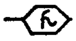 | 372 |
| Standard | $\Phi_3P$ | 510 |

[a] Purchased from Strem Chemicals Inc., Newburyport, Mass.
[b] Purchased from Organometallics Inc., East Hampstead, N. H.
[c] Prepared by reacting lithium diphenyl phosphide with the corresponding alkyl chloride.

Example 13
Preparation of Neopentyl diphenyl phosphine

$$Ph_2PLi+ClCH_2C(CH_3)_3 \rightarrow Ph_2PCH_2C(CH_3)_3$$

The known but unavailable 2,2-dimethylpropyl diphenyl phosphine was derived via reacting 2,2-dimethylpropyl chloride with lithium diphenyl phosphide in a refluxing tetrahydrofuran-hexane solvent mixture. After filtering off the lithium chloride by-product, the 2,2-dimethylpropyl, (i.e., neopentyl) diphenyl phosphine was obtained by the fractional distillation of the filtrate between 109 and 110°C at 0.1 mm.

Example 14
Preparation of 3,3-dimethylbutyl diphenyl phosphine

$$Ph_2PH+CH_2{=}CHC(CH_3)_3 \rightarrow Ph_2PCH_2CH_2C(CH_3)_3$$

Diphenyl phosphine and t-butylethylene, i.e. 3,3-dimethyl butene, were reacted in the manner of Example 1. However, some phase separation occurred, and consequently, the reaction was slow. The expected adduct was separated from the reactants by fractional distillation. It was obtained as a colorless, clear liquid, boiling between 125—127°C at 0.2 mm.

As a known compound, t-butylethyl, (i.e., 3,3-dimethylbutyl) diphenyl phosphine was also synthesized via the known displacement approach: the reaction of lithium diphenyl phosphide with 3,3-dimethylbutyl chloride provided the compound in good yield.

Alkylene bis-(diphenyl phosphines)—(Examples 16—22)

As a class of compounds alkylene bis-diphenyl phosphines are known. In the present work, available compounds were used. The bis-phosphines as such were studied only to determine their basicity. The results are shown in Table 2. According to the results at the transition from chelating to non-chelating phosphines (n=3 to n=4) different phosphine species are present as indicated by the pairs of HNP values.

TABLE 2
Alkylene bis-(diphenyl phosphine) ligands and their basicity
$Ph_2P(CH_2)_mPPh_2$

| Example | Polymethylene bridge, m | Indirect basicity ΔHNP |
|---|---|---|
| 16 | 1 | 453 |
| 17 | 2 | 431 |
| 18 | 3 | 395, 548 |
| 19 | 4 | 315, 378 |
| 20 | 5 | — |
| 21 | 6 | 423 |
| 22 | 14 | — |

Variously substituted alkyl diphenyl phosphines (Examples 23—47)

All the ligands which will be subsequently described were prepared by the addition of diphenyl phosphine to differently substituted vinylic compounds. Most of the adducts are novel, and none of them were previously prepared by the present method.

Example 23
Preparation of phenylethyl diphenyl phosphine

$$Ph_2PH+CH_2{=}CHPh \rightarrow Ph_2PCH_2CH_2Ph$$

2-Phenylethyl diphenyl phosphine, a known but unavailable compound, was prepared in the present work via a new method, i.e., the addition of diphenyl phosphine to styrene. A mixture of the unsaturated compound (20.8 g, 0.20 mole) and the phosphine (39.1 g, 0.21 mole, 5% excess) was irradiated for 5 hours in the usual manner. The desired adduct, 2-phenylethyl diphenyl phosphine, was obtained as a pure colorless, clear liquid distillate by distillation in vacuo. The distillation yield was 87% and the product had a boiling point of 171—173°C at 1 mm. (Table 3).

Example 24
Preparation of pyrrolidinonylethyl diphenyl phosphine

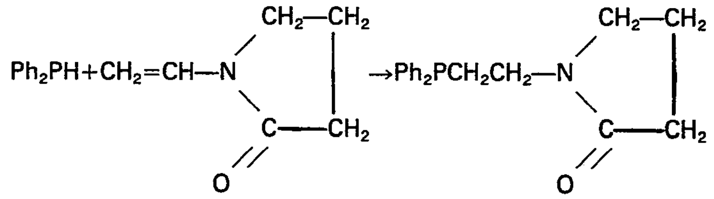

A mixture of 37.2 (0.2 mole) diphenyl phosphine and 22.2 g (0.2 mole) N-vinyl-2-pyrrolidinone was reacted with U.V. initiation for 48 hours. GLC analyses of the reaction mixture indicated that after 5, 24 and 48 hours the conversions to the desired adduct were 63, 93 and 99% respectively. The crude product was purified by distillation to obtain pure N-2-pyrrolidinonylethyl diphenyl phosphine as a hazy, colorless, viscous liquid (Table 3).

TABLE 3

Preparation, physical properties and composition of various substituted alkyl diphenyl phosphine ligands

| Example No. | Example No. E— | Structure of ligand | Unsaturated reactant used | Ligand op. °C/mm (hp. °C) | Distd yield ~% | Elemental composition Calcd. C | H | P | Found C | H | P | Inverse basicity ΔHNP |
|---|---|---|---|---|---|---|---|---|---|---|---|---|
| 23 | 4275—VI | $Ph_2PCH_2CH_2Ph$ | $CH_2=CHPh$ | 171—173/0.1 | 87 | 82.74 | 6.60 | 10.66 | 82.30 | 6.67 | 10.77 | 416 |
| 24 | 5382—IX | 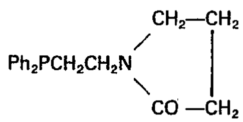 | 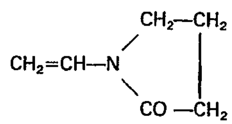 | 181—178/0.1 | 83 | 72.71 | 6.89 | 10.42 | 72.31 | 6.71 | 10.50 | 450 |
| 25 | 5433—IX | $Ph_2PCH_2CH_2CH_2H(C_2H_5)_2$ | $CH_2=CHCH_2H(C_2H_5)_2$ | 137—138/0.1 | — | 76.22 | 8.75 | 4.68 | 76.55 | 8.76 | | |
| 26 | 5431—VIII | $Ph_2PCH_2CH_2SO_2C_2H_5$ | $CH_2=CHSO_2C_2H_5$ | [b] | 69 | 62.73 | 6.25 | 10.11 | 62.49 | 6.17 | | 543 |
| 27 | 5421—III | $Ph_2PCH_2CH_2POPh_2$ | $CH_2=CHPOPh_2$ | (182—184[a]) | 41[a] | 75.36 | 5.84 | 14.95 | 74.85 | 5.84 | 14.52 | 430 |
| 28 | 5391—XII | $Ph_2PCH_2CH_2COCH_3$ | $CH_2=CHCOCH_3$ | 148—150/ 0.09—0.08 | 66 | 74.99 | 6.69 | 12.08 | 74.53 | 6.58 | 11.82 | 425 |
| 29 | 5384—X | $Ph_2PCH_2CH_2CO_2CH_3$ | $CH_2=CHCO_2CH_3$ | 162—160/ 0.30—0.32 | 60 | 70.58 | 6.29 | 11.38 | 70.81 | 6.31 | 11.25 | 455 |

[a] Recrystallized from methanol

[b] Recrystallized from 1-propanol which was also used as the solvent for the reaction.

Example 25
Preparation of diethylaminopropyl diphenyl phosphine

$$Ph_2PH+CH_2{=}CHCH_2N(C_2H_5)_2 \rightarrow Ph_2PCH_2CH_2CH_2N(C_2H_5)_2$$

A mixture of 17 g (0.15 mole) allyl diethyl amine and 30.7 g (0.165 mole, 10% excess) of diphenyl phosphine is reacted in the usual manner with u.v. irradiation for 17 hours. A subsequent analysis of the reaction mixture indicated that about one third of the reactants was converted. The only product formed was the desired adduct, 2-diethylaminopropyl diphenylphosphine. It was isolated as a clear colorless liquid (Table 3).

Example 26
Preparation of Ethylsulfonylethyl diphenyl phosphine

$$Ph_2PH+CH_2{=}CHSO_2C_2H_5 \rightarrow Ph_2PCH_2CH_2SO_2C_2H_5$$

A mixture of 64 g (0.34 mole) of diphenyl phosphine and 40.2 g (0.33 mole) of highly reactive, freshly distilled vinyl ethyl sulfone monomer was irradiated in the usual manner. To suppress polymer forming side reactions the temperature of the reaction mixture was kept below 5°C by an ice-water bath and the u.v. irradiation was limited to 105 minutes. The adduct formed crystallized from the liquid mixture by the end of the reaction period. Consequently, the reaction mixture was filtered with suction to recover the crude, crystalline product, i.e. 2-ethyl-sulfonylethyl diphenyl phosphine. The crude, dried product (94 g, 94%) was recrystallized from 670 ml methanol to yield 69 g (69%) of the compound (of unpleasant odor) as a white crystalline solid (Table 3).

Example 27
Preparation of diphenylphosphinoethyl diphenyl phosphine oxide

$$Ph_2PH \xrightarrow{O_2} Ph_2\overset{O}{\overset{\|}{P}}H \xrightarrow{Ph_2PCH=CH_2} Ph_2PCH_2CH_2\overset{O}{\overset{\|}{P}}Ph_2$$

2-Diphenylphosphinoethyl diphenyl phosphine oxide, a known, but unavailable compound, was prepared via a new approach. In the first step, diphenyl phosphine was quantitatively air oxidized at 60°C in isopropanol solution. The resulting diphenyl phosphine oxide was then added to vinyl diphenyl oxide in the manner of Example 1, with u.v. initiation.

A 26.5 wt% by wt isopropanol solution of 33.9 g (0.16 mole) of the phosphine oxide adding agent and 34.1 g (0.16 mole) of the vinyl phosphine reagent were mixed. The resulting homogeneous liquid mixture was irradiated with stirring at 30°C for 66 hours. By the end of the reaction period, the adduct formed crystallized from the mixture. Subsequently, the crude product was dissolved in refluxing isopropanol after adding an additional 124 g of the solvent. A glc analysis of the hot solution showed that the desired addition was complete and no side reaction occurred. Consequently, the solution was allowed to cool. This resulted in the crystallization of the product. The latter was separated by filtration with suction and washing with cold isopropanol. Subsequent drying in vacuo provided 66 g (41%) of the pure compound as a white crystalline solid (Table 3).

Example 28
Preparation of acetylethyl diphenyl phosphine

$$Ph_2PH+CH_2{=}CHCOC_2H_5 \rightarrow Ph_2PCH_2CH_2COC_2H_5$$

$$+Ph_2PCH_2\underset{\displaystyle CH_2CH_2COC_2H_5}{\underset{|}{C}}HCOC_2H_5$$

To 37.2 g (0.2 mole) stirred, nitrogen blanketed diphenyl phosphine, 15.2 g (0.22 mole) of the highly reactive vinyl ethyl ketone reactant was added dropwise. During the addition an exothermic reaction took place. As a consequence, the temperature of the reaction mixture rose to 50°C. A subsequent GLC analysis indicated that the vinyl reactant was mostly converted. Major amounts of the desired adduct and minor amounts of the diadduct were formed. The adduct, i.e. 2-acetylethyl diphenyl phosphine, was obtained by fractional distillation of the reaction mixture. At room temperature, it solidified to a light yellow substance (Table 3).

Example 29
Preparation of carbomethoxyethyl diphenyl phosphine

$$Ph_2PH+CH_2{=}CHCO_2CH_3 \rightarrow Ph_2PCH_2CH_2CO_2CH_3$$

$$+Ph_2PCH_2CHCO_2CH_3$$
|
$$CH_2CH_2COCH_3$$

A mixture of 8.6 g (0.1 mole) methyl acrylate and 19.5 g (0.105 mole, 5% excess) of diphenyl phosphine was reacted at 15°C in the routine manner of Example 1. U. V. irradiation resulted in a rapid reaction. After 1 hour, there was no acrylate left unconverted. The expected mono- and diadducts were formed in a weight ratio of about 95 to 5. (A sample of the reaction mixture which was not irradiated also showed a complete conversion after 22 hours but not after 1 hour). On distillation of the reaction mixture in vacuo, the monoadduct, i.e. 2-carbomethoxyethyl diphenyl phosphine, was obtained as an almost colorless, liquid distillate having a slight yellow tint (Table 3).

Example 30
Preparation of hydroxypropyl diphenyl phosphine

$$Ph_2PH+CH_2{=}CHCH_2OH \rightarrow Ph_2PCH_2CH_2CH_2OH$$

A mixture of 11.5 g (0.062 mole) diphenyl phosphine and 3.6 g (0.062 mole) of allyl alcohol was reacted with irradiation initiation at 15°C for 110 hours in the usual manner. Subsequent glc and nmr analyses indicated complete conversion to the desired adduct, i.e. 3-hydroxypropyl diphenyl phosphine. Distillation in vacuo provided 10.5 g (72%) of the pure compound, between 162—164°C at 0.15 mm, as a clear colorless liquid.

Anal. Calcd. for $C_{15}H_{17}OP$: C, 73.76; H, 7.01; P, 12.68. Found: C, 73.52; H, 6.89; P, 12.82.

Example 31
Preparation of bis-(diphenylphosphinopropyl) ether

$$2\ Ph_2PH+(CH_2{=}CHCH_2)_2O \rightarrow (Ph_2PCH_2CH_2CH_2)_2O$$

A mixture of 7.3 g (0.75 mole) diallyl ether and 30.7 g (0.165 mole, 10% excess over the mole equivalent amount) was irradiated at 15°C for 3 days to effect the desired addition. Glc showed that the conversion of diphenyl phosphine was about 66%. The major product, about 50% of the mixture was the desired diadduct, bis-(3-diphenylphosphinopropyl) ether. The pure diadduct was obtained by distillation in vacuo.

Example 32
Preparation of methylthiopropyl diphenyl phosphine

$$Ph_2PH+CH_2{=}CHCH_2SCH_3 \rightarrow Ph_2PCH_2CH_2CH_2SCH_3$$

A mixture of 10.6 g (0.12 mole) of allyl methyl ether and 24.6 g (0.132 mole, 10% excess) diphenyl phosphine was reacted at 15°C with u.v. initiation for 28 hours. Glc indicated an essentially complete but nonselective conversion of the reactants. The selectivity to the desired adduct, 3-methylthiopropyl diphenyl phosphine, was about 50%. On distillation in vacuo 13 g (40%) of the compound was obtained as a clear, colorless liquid, bp. 150—152°C at 0.2 mm.

Anal. Calcd. for $C_{14}H_{19}PS$: C, 70.04; H, 6.98; P, 11.29; S, 11.69. Found: C, 69.78; H, 6.82; P, 11.35; S, 11.93.

Examples 33—47
Miscellaneous substituted alkyl diaryl phosphines

Using the methods described in the previous examples, diphenyl phosphine can be added to a number of vinylic and allylic compounds to yield the corresponding anti-Markovnikov adducts, i.e. substituted alkyl diphenyl phosphines, as shown by the following tabulation.

| Example No. | Unsaturated reagent for diphenyl phosphine | Substituted alkyl diphenyl phosphine |
|---|---|---|
| 33 | Vinyl naphthalene | Naphthyl ethyl diphenyl phosphine |
| 34 | Allyl amine | Aminopropyl diphenyl phosphine |
| 35 | Allyl morpholine | Morpholinopropyl diphenyl phosphine |
| 36 | Acrylamide | Carbamylethyl diphenyl phosphine |
| 37 | Vinyl carbazole | Carbazylethyl diphenyl phosphine |
| 38 | Vinyl pyridine | Pyridylethyl diphenyl phosphine |
| 39 | Vinyl phthalimide | Phthalimidoethyl diphenyl phosphine |
| 40 | Vinyl diethyl phosphonate | Diethyoxyphosphonylethyl diphenyl phosphine |
| 41 | Allyl ethyl ether | Ethoxypropyl diphenyl phosphine |
| 42 | Vinyl isopropyl ether | i-Propoxyethyl diphenyl phosphine |
| 43 | Vinyl furan | Furylethyl diphenyl phosphine |
| 44 | Allyl acetate | Acetoxypropyl diphenyl phosphine |
| 45 | Vinyl benzoate | Benzoyloxyethyl diphenyl phosphine |
| 46 | Allyl phenyl sulfide | Phenylthiopropyl diphenyl phosphine |
| 47 | Divinyl sulfone | Bis-(diphenylphosphinoethyl) sulfone |

Similar additions are carried out using di-4-tolyl phosphine and difluorophenyl phosphine and the above unsaturated reactants to yield the corresponding ring substituted products.

Preparation and properties of tris-(alkyl diaryl phosphine) rhodium carbonyl hydride complexes
Preparation from rhodium chloride
Example 48
Preparation of tris-(trimethylsilylethyl diphenyl phosphine) rhodium carbonyl hydride

$$3(Ph_2PCH_2CH_2Si(CH_3)_3 + RhCl_3 \, . \, 3H_2O \rightarrow [Ph_2PCH_2CH_2Si(CH_3)_3]_3Rh(CO)H$$

To a vigorously stirred, refluxing, nitrogenated solution of 11.44 g (40 mmole) of trimethylsilylethyl diphenyl phosphine of Example 1 in 400 ml of ethanol, a hot solution of 1.04 g (0.4 mmole) of rhodium trichloride trihydrate in 80 ml ethanol was added at once. After a delay of 15 seconds, 40 ml warm aqueous (37%) formaldehyde solution and, immediately thereafter, 80 ml hot ethanolic solution of 3.2 g of potassium hydroxide were added. The resulting clear orange liquid reaction mixture was refluxed for 10 minutes. During the heating, the color changed to deep orange.

The mixture was cooled to −25°C to crystallize the complex product. Crystallization started at −10°C and was completed on standing for about 2 hours at −25°C. The crystalline complex was separated by filtration through a precooled Buechner funnel with suction and washing successively with 20 ml cold portions of ethanol, water, ethanol and n-hexane. The complex was then dried in the presence of anhydrous calcium chloride at 9.1 mm over the weekend. As a result, 2.2 g (2.2 mmole, 55%) of dry tris-(trimethylsilylethyl diphenyl phosphine) rhodium carbonyl hydride complex was obtained as a fine crystalline orange-yellow powder. In a sealed capillary tube, the complex melted between 126—129°C to a clear dark red liquid. In an open capillary, complete melting occurred at 121°C. There was no sign of decomposition on heating up to 140° in either case.

The infrared spectrum of the complex in Nujol showed a strong carbonyl band of 1985 $cm^{-1}$ and a band of medium intensity at 1900 $cm^{-1}$.

Analyses Calcd. for $C_{52}H_{70}OP_3RhS$: C, 63.01; H, 7.12; P, 9.38; Found: C, 62.89; H, 7.06; P, 9.59.

Preparation from tris-(triphenyl phosphine) rhodium
Carbonyl hydride via ligand displacement

$$(Ph_3P)_3Rh(CO)H+3Ph_2PR \rightarrow (Ph_2PR)_3Rh(CO)H+3Ph_3P$$

The tris-(alkyl diaryl phosphine) rhodium carbonyl hydride complexes were prepared by reacting the readily available tris-(triphenyl phosphine) rhodium carbonyl hydride (from Engelhard Minerals and Chemicals Corporation, Newark, N. J.) with the corresponding alkyl diaryl phosphines. Generally, the reactions were performed in a mixture of toluene and deuterated benzene as a solvent under a nitrogen blanket. The deuterated benzene component was used as a primary nmr standard.

At first, a solution of about 5% of the alkyl diaryl phosphine reactant was prepared. To samples of the solution, TPP rhodium carbonyl hydride was added in equivalent and half equivalent amounts. The resulting mixtures were magnetically stirred until homogeneous liquids were obtained. Additional amounts of the toluene solvent were used if needed. The homogeneous reaction mixture was then studied by $^{31}P$ nmr spectroscopy. Chemical shifts were measured by assigning a shift of O ppm to the frequency at which 1M $H_3PO_4$ would resonate.

The $^{31}P$ nmr experiments were carried out using a JEOL FX 90Q multi-nuclear nmr spectrometer. When required the experimental conditions were adjusted, i.e. the $^1H$—$^{31}P$ decoupling was removed and longer delays between pulses were employed, to determine the relative populations of free and rhodium bound alkyl diphenyl phosphine and TPP.

The $^{31}P$ nmr experiments are illustrated by Figure 2 which shows the spectrum of a six to one mixture of n-butyl diphenyl phosphine and tris-TPP rhodium carbonyl hydride, in comparison with a spectrum of a three to one mixture of TPP and tris-TPP rhodium carbonyl hydride.

In the presence of excess butyl diphenylphosphine, the only significant doublet peak of complexed phosphorus is that derived from butyl diphenyl phosphine. This complex was derived by ligand exchange as indicated by the singlet of displaced free TPP. Although the doublet of the butyl diphenyl phosphine complex has a chemical shift value different from that of TPP, the coupling constants are about the same for both complexes. The coupling constant and chemical shift difference between bound and free ligand indicate that both ligands form tris-(phosphine) rhodium carbonyl hydrides.

Similar ligand exchange experiments were carried out with other alkyl diphenyl phosphines to form their tris-(phosphine) rhodium carbonyl hydride complexes to determine the characteristic nmr parameters of such complexes.

The nmr parameters of the trihydrocarbylsilylalkyl diphenyl phosphine complexes are shown by Table 4. The most characteristic parameter is the chemical shift value of the rhodium complexed ligand. For comparison, the chemical shift values of the free ligands are also tabulated. Complexation by rhodium of the phosphine apparently produced a similar downfield change of the shift values. Finally, it is also noted in reference to the table, that even the limited exposure of the rhodium complexed phosphines to air resulted in some oxidation to the corresponding phosphine oxides. The latter exhibited sharp singlets slightly upfield from the complexed phosphine.

The data of Table 4 shows that with the exception of the last compound all the phosphine ligands form similar well characterizable complexes at room temperature. The line shapes of the signals showed little but varying broadening, i.e., ligand exchange. In the case of the tris-(trimethylsilylethyl diphenyl phosphine), tris-SEP, rhodium carbonyl hydride complex (Example 48), there was moderately slow ligand exchange between free and complexed phosphines. The exchange mechanism is illustrated for the SEP complex by the following (for details of the hydroformylation mechanism refer to Figure 1):

$$(SEP)_3Rh(CO)H \underset{+SEP}{\overset{-SEP}{\rightleftarrows}} (SEP)_2Rh(CO)H \underset{-SEP}{\overset{+SEP^*}{\rightleftarrows}} (SEP)_2SEP^*Rh(CO)H$$

The line shapes of signals for the SEP complex and the known TPP complex are compared by Figure 3 at various temperatures. At first, the 30°C spectra will be discussed. These spectra indicate that at 30°C, there is a similar, ligand exchange rate between the new SEP and the known TPP complex.

TABLE 4

$^{31}P$ Nuclear magnetic resonance parameter of free and rhodium-complexed trihydrocarbylsilylalkyl diphenyl phosphines

| Example No. of complex | Example No. of ligand | Chemical structure of complex | Chemical shift 5, ppm | | Coupling constant P—Rh complexed ligand | Chemical shift ppm phosphine oxide | Chemical shift differences ppm complex ligand |
|---|---|---|---|---|---|---|---|
| | | | Free ligand | Complexed ligand | | | |
| — | — | $(Ph_3P)_3Rh(CO)H$ | −7.5 | +38.3 | 155 | | 45.8 |
| 48 | 1 | $[Ph_2PCH_2CH_2Si(CH_3)_3]_3Rh(CO)H$ | −12.1 | +34.6 | 150 | +27.0 | 46.7 |
| 49 | 2 | $[Ph_2PCH_2CH_2Si(C_3H_7)_3]_3Rh(CO)H$ | −11.2 | +34.8 | 151 | +29.9 | 45.0 |
| 50 | 3 | $(PH_2PCH_2CH_2SiPh_3)_3Rh(CO)H$ | −10.6 | +35.5 | 151 | +21.1 | 46.1 |
| 51 | 4 | $[(Ph_2PCH_2CH_2)_2Si(CH_3)_2]_3[Rh(CO)H]$ | −12.2 | | | | |
| 52 | 5 | $[Ph_2PCH_2CH_2CH_2Si(CH_3)_3]_3Rh(CO)H$ | −19.7 | +26.6 | 154 | +24.6 | 46.3 |
| 53 | 6 | $[Ph_2PCH_2Si(CH_3)_3]_3Rh(CO)H$ | −24.4 | +17.0 | 133[a] | +23.9 | 41.4 |

[a] The $^{31}P$—$^{103}Rh$ coupling was not resolved at room temperature but was clearly resolved at 60°C.

The tris-SEP complex and two other trimethylsilylalkyl diphenyl phosphine complexes (Examples 51 and 52) showed a very similar ligand exchange behavior at 30°C. The tripropylsilylethyl diphenyl phosphine complex (Example 49) exhibited a definitely slower exchange rate. The exchange rate of the triphenylsilylethyl diphenyl phosphine complex (Example 50) was even much slower than that. It appeared that substituted alkyl diphenyl phosphine ligands of increasing bulkiness had decreasing ligand exchange rates. In both cases though, the TPP ligand exchanged less rapidly than the alkyl diphenyl phosphine.

Finally, it is noted that when even a moderately bulky alkyl substituent was close to the phosphorus, i.e., in the case of trimethylsilylmethyl diphenyl phosphine, the complexation of phosphorus to the rhodium was inhibited (Example 54). In that case, there was no distinct complex formation with the sterically hindered ligand at 30°C. AT −60°C, a stable complex was formed. However, this complex was decomposed when its solution was heated under hydroformylation process conditions.

As far as ligand exchange rates at higher temperatures are concerned, the results shown by Figure 3 are typical. Figure 3 shows the comparison of two systems: tris-triphenyl phosphine rhodium carbonyl hydride plus triphenyl phosphine and tris(trimethylsilylethyl diphenyl phosphine) rhodium carbonyl hydride plus triphenyl phosphine. The latter system is the result of equilibrating the TPP complex with trimethylsilylethyl diphenyl phosphine (SEP):

$$(Ph_3P)_3Rh(CO)H + 3Ph_2PCH_2CH_2SiMe_3 \rightarrow [Ph_2PCH_2CH_2SiMe_3]_3Rh(CO)H + 3Ph_3P$$

SEP being a substituted alkyl diphenyl phosphine, was found to be a stronger complexing agent than TPP. The spectra of both systems were taken under comparative conditions at 30, 60 and 90°.

The line shapes of the signals of the 30°C spectra showed little signal broadening in both cases. This indicated comparably slow exchange rates of about 25 per second. In alternative terms, relatively long average exchange lifetimes, in the order of $2\times10^{-2}$ sec, were indicated for both tris-phosphine complexes. At 60°, considerable line broadening occurred, indicating a much faster exchange. The exchange acceleration was greater in the case of the TPP system (k=600 vs. 80). The average lifetime was about $3\times10^{-3}$ sec for the TPP system and $6\times10^{-3}$ sec for the SEP system. At 90°, only a single, broad signal could be observed for the TPP system while the SEP system still exhibited separate, although extremely broad, chemical shift ranges for the complexed and free phosphorus species. Apparently, the exchange acceleration in the case of the TPP system was tremendous. The average lifetime between exchanges was reduced about two orders of magnitude to $5\times10^{-5}$ sec (k≃10,000). In the case of the SEP system, the average lifetime dropped by about one order to $5\times10^{-4}$ sec (k≃1,500). It must be emphasized that the exchange rates and lifetimes reported here may change somewhat when the lineshape is subjected to a rigorous computer analysis. The relative order of their values will remain unaltered, however.

It is interesting to note that there was no great change of equilibria with the increasing exchange rates. Apparently, both ligand elimination and addition increase similarly in this temperature range. The tris-phosphine rhodium species remained the dominant form of complexes. In the SEP complex plus free TPP system, the rhodium remained predominantly complexed to the SEP.

The role of excess phosphine ligand is apparently to maintain the equilibria in favor of the tris-phosphine complex, i.e. to reduce both the concentration and average lifetime of the unstable and highly reactive bis-phosphine complex. The increased ligand exchange rate provides enough active bis-phosphine complex catalytic species for fast hydroformylation, without leading to noncatalytic side reactions, i.e. catalyst decomposition.

In summary, the above and similar ligand exchange rate studies indicate that, in the presence of excess ligand, the alkyl diaryl phosphine rhodium complexes are catalytically activated at higher temperatures than the known triaryl phosphine rhodium complexes.

The results of a similar systematic $^{31}P$ study of various alkyl diphenyl phosphines is summarized in Table 5. The Table shows the $^{31}P$ nmr parameters of free and rhodium complexed alkyl diphenyl phosphines in solution at 35°. An overview of Table 5 indicates that five of the seven phosphine ligands examined formed tris-phosphine rhodium carbonyl hydride complexes. Steric crowding apparently inhibited complex formation. Comments on the detailed data of the table are made in the following.

As it was already discussed in conjunction with Figure 2, n-butyl diphenyl phosphine exhibits a ligand exchange behavior similar to that of the SEP ligand (Example 54). However, it was slightly less effective in completely displacing TPP. In the latter respect, the bulkier n-hexyl diphenyl phosphine was a more effective ligand (Example 55).

In comparison to n-butyl diphenyl phosphine, secondary butyl diphenyl phosphine is quite ineffective in replacing the TPP ligand (Example 56). Cooling to −60° was necessary to observe a clearly resolved doublet signal for the complexed secondary butyl compound. Alternatively, a complex of this ligand could be obtained at room temperature starting with $(PH_3As)_3Rh(CO)H$ (TPA complex) in place of the TPP complex.

TABLE 5

$^{31}P$ Nuclear magnetic resonance parameters of free and rhodium complexed alkyl diphenyl phosphines

| Example No. of complex | Example No. of ligand | Chemical structure of complex | Chemical shift σ ppm Free ligand | Chemical shift σ ppm Complexed ligand | Coupling constant JP—Rh complexed ligand | Chemical shift difference Δσ ppm complex ligand |
|---|---|---|---|---|---|---|
| 54 | 9 | $(\phi PCH_2CH_2CH_2CH_3)_3Rh(CO)H$ | −18.6 | +27.4 | 149 | 46.0 |
| 55 | 10 | $(\phi PCH_2CH_2CH_2CH_2CH_2CH_3)_3Rh(CO)H$ | | | | |
| 56 | 11 | $(O_2PCHCH_2CH_3)_3Rh(CO)H$<br>\|<br>$CH_3$ | −4.8 | +40.0 | 154[a] | 45.0 |
| 57 | 12 | $[\phi_2PC(CH_3)_3]_3$ | +15.6 | | | |
| 58 | 13 | $[\phi_2PCH_2C(CH_3)_3]_3$ | −25.1 | | | |
| 59 | 14 | $[\phi_2PCH_2CH_2C(CH_3)_3]_3Rh(CO)H$ | −16.8 | +27.5 | 152 | 44.3 |

[a] The $^{31}P$—$^{103}Rh$ coupling was not resolved at room temperature but was clearly resolved at −60°C.

Tertiary butyl and neopentyl diphenyl phosphine (Examples 57 and 58) did not form tris-phosphine complexes under standard experimental conditions. Surprisingly, the t-butyl compound had a destabilizing effect on the TPP complex reactant. As a result the mixture rapidly turned black, apparently due to rhodium precipitation.

3,3-Dimethylbutyl diphenyl phosphine exhibited a complex forming and equilibration tendencies similar to those of its silicon analog, SEP.

Finally, it is noted that cyclohexyl diphenyl phosphine only partially replaced TPP from its complex and exhibited a very high rate of ligand exchange (Example 60). Overall this ligand and the secondary butyl diphenyl phosphine had a comparable complexing behavior. In both cases, steric crowding was a severely limiting factor.

Other suitable substituted alkyl diaryl phosphines are reacted similarly with tris-(triphenyl phosphine) rhodium carbonyl hydride, tris-(triphenyl arsine) rhodium carbonyl hydride and the like to provide the corresponding tris-phosphine complexes. For example, starting with the substituted alkyl diphenyl phosphine ligands of Examples 33 to 47, the following miscellaneous substituted alkyl diphenyl phosphine complexes are formed:

| Example No. 5 | | |
|---|---|---|
| Complex | Ligand | Name of the complex formed |
| 60 | 33 | tris-(naphthyl ethyl diphenyl phosphine) rhodium carbonyl hydride |
| 61 | 34 | tris-(aminopropyl diphenyl phosphine) rhodium carbonyl hydride |
| 62 | 35 | tris-(morpholinopropyl diphenyl phosphine) rhodium carbonyl hydride |
| 63 | 36 | tris-(carbamylethyl diphenyl phosphine) rhodium carbonyl hydride |
| 64 | 37 | tris-(carbazylethyl diphenyl phosphine) rhodium carbonyl hydride |
| 65 | 38 | tris-(pyridylethyl diphenyl phosphine) rhodium carbonyl hydride |
| 66 | 39 | tris-(phthalimidoethyl diphenyl phosphine) rhodium carbonyl hydride |
| 67 | 40 | tris-(diethyoxyphosphonylethyl diphenyl phosphine) rhodium carbonyl hydride |
| 68 | 41 | tris-(ethoxypropyl diphenyl phosphine) rhodium carbonyl hydride |
| 69 | 42 | tris-(2-propoxyethyl diphenyl phosphine) rhodium carbonyl hydride |
| 70 | 43 | tris-(furylethyl diphenyl phosphine) rhodium carbonyl hydride |
| 71 | 44 | tris-(acetoxypropyl diphenyl phosphine) rhodium carbonyl hydride |
| 72 | 45 | tris-(benzoyloxyethyl diphenyl phosphine) rhodium carbonyl hydride |
| 73 | 46 | tris-(phenylthiopropyl diphenyl phosphine) rhodium carbonyl hydride |
| 74 | 47 | tris-[bis-(diphenylphosphinoethyl) sulfone] bis-(rhodium carbonyl hydride) |

Similar substituted alkyl diaryl phosphine complexes are prepared from substituted alkyl difluorophenyl phosphines, substituted alkyl ditolyl phosphines, and substituted alkyl phenyl naphthyl phosphines.

By selecting the appropriate substituent, the complex catalysts of the present invention can be fine tuned to provide optimum performance at the desired temperature. Also, substitution could be used as a means of adjusting the solubility character of the free and complexed alkyl diaryl phosphine ligands. For example, the hydrophilic-lipophilic character of the ligand could be appropriately changed by introducing either large hydrocarbon substituents (Example 3) or highly polar groups (Example 24). The acid-base character can be also changed. For example, a basic amino group could be introduced (Example 25). Such a group can be the essential factor in catalyst recovery. Other ligand substituents can increase the solubility of gaseous reactants such as CO in the liquid reaction medium. A multiplicity of non-chelating phosphine groups will drastically reduce ligand volatility.

In general, it should be pointed out that a correlation of the nmr and catalysis studies showed that those complexes which show lower ligand exchange rates at low temperatures have a higher activation energy as catalyst. That means that they required more thermal activation, i.e. higher temperatures, to become highly active catalysts. Less ligand exchange also meant a higher temperature for the irreversible thermal dissociation, i.e. decomposition of the catalyst complex.

Under the preferred process conditions for the present catalysts, the structure of the tris-(alkyl diphenyl phosphine) rhodium carbonyl hydrides as described by the nmr parameters could be formed in situ and did not undergo any irreversible change. For example, the tris-SEP complex was generated under routine hydroformylation conditions at 120° from dicarbonyl acetylacetonato rhodium and was employed for 1-butene hydroformylation. After the reaction was complete, the volatile components were removed by distillation and the residual liquid was studied by $^{31}P$ nmr. The typical parameters for tris-SEP rhodium carbonyl hydride and excess free SEP were found.

General method of hydroformylation

The hydroformylation of butene-1 to provide linear pentanal and branched 2-methyl butanal products was selected for comparative studies of the catalytic properties of certain of the alkyl diaryl phosphine complexes of the invention. The complexes studied were either isolated before use or generated *in situ*. In some cases, the desired complex was generated from the known tris-(triphenyl phosphine) rhodium carbonyl hydride by the addition of the appropriate ligand in varying amounts. According to another standard method, dicarbonyl acetylacetonato rhodium and the appropriate alkyl diaryl phosphine were used as catalyst precursors. In that case, the desired rhodium carbonyl hydride complex was generated by hydrogenation during the hydroformylation experiment. Tris-(triphenyl phosphine) rhodium carbonyl hydride in the presence of varying excesses of triphenyl phosphine was used as a known catalyst standard for comparison.

The experiments were carried out in a 300 ml stainless steel (S) and a 300 ml Hastelloy (H) autoclave, respectively. Both autoclaves were equipped with identical, highly effective, impeller type stirrers, operating at 750 rpm during the experimental runs. The other standard autoclave instrumentation was identical for both units. However, a slightly lower normal to iso aldehyde product ratio (n/i) was observed in unit H. In those cases where the type of autoclave was not specified, a stainless steel unit was used.

The standard batch hydroformylation procedure was the following: the appropriate amounts of rhodium complex were dissolved in 100 g of the proper mixture of a free phosphine and solvent. 2-propylheptyl valerate or 2-ethylhexyl acetate were used as standard solvents. They were indistinguishable. Most often the amount of complex employed provided 100 ppm rhodium concentration. This meant 100 mg, i.e., about 0.1 mmole rhodium per 100 g. Accordingly, 100 mg per Kg, about 1 mmole per kg rhodium would be present in 1 kg starting mixture. The excess ligand added to the solvent was usually calculated to provide a ligand to rhodium ratio (L/Rh) of about 140.

The 100 g rhodium complex-ligand solution was placed into the autoclave which was then deaerated by repeated pressurization with nitrogen. The solution under atmospheric nitrogen pressure was then sealed and heated to the reaction temperature, usually 100°C.

When the solution reached 100°C, 20 g of liquid butene was pressured into the autoclave with a 1 to 4 carbon monoxide-hydrogen initial gas mixture. The butene was followed by the $CO/H_2$ mixture until a pressure of 350 psig was reached. At that point, the supply of 1:4 $CO/H_2$ was shut off and the autoclave was connected to a cylinder of about 1 liter volume containing a 1:1 $CO/H_2$ feed gas mixture at 1000 psig. The connection was made through a pressure regulating valve set to provide the 1:1 $CO/H_2$ gas to the autoclave to maintain a 350 psig pressure during the reaction. The exact $H_2/CO$ ratio of the feed gas was often varied to maintain the initial $H_2/CO$ ratio in the autoclave.

In the standard tests, the autoclaves used were equipped with synthesis gas feed lines adjoining the autoclave above the Magnedrive stirrer assembly unit (Figure 4). It is to be noted that this manner of introducing synthesis gas feed far from the upper level of the liquid reaction mixture resulted in an incomplete equilibriation of the synthesis gas mixture between the gas and liquid phase. Particularly in those cases where the initial synthesis gas mixture (used to pressure up the reaction mixture) had an $H_2$ to CO ratio of 10 or higher, the CO component of the subsequent one to one feed gas was not effectively delivered from the top into the liquid reaction mixture due to mass transfer limitations. Therefore, the reaction mixture was often "starved" of CO during the early fast phase of the reaction. As a consequence,

the $H_2$/CO ratio in the liquid temporarily rose to very high values. This resulted in particularly high n- to i-aldehyde product ratios. Also, olefin hydrogenation and isomerization became important side reactions. This, of course, reduced the absolute accuracy of the data on catalyst selectivities. Nevertheless, in a relative sense, the data are correct in all cases. For comparison, the widely studied Tris-TPP rhodium carbonyl hydride catalyst system was used as a standard throughout the work.

In those instances, where the effect of $H_2$ to CO ratios and the effect of CO partial pressure were specifically studied, the synthesis gas feed was introduced at the side of the autoclave, just above the liquid level. This method of operation largely avoided any temporary rise $H_2$/CO ratios and drastically reduced hydrogenation and isomerization in cases where the initial $H_2$/CO ratio was high. Special studies were also made in a continuous feed introduction and product flashoff operation. This allowed a continuous control of partial pressures and such provided the most accurate results (Figure 12).

The progress of the hydroformylation was followed on the basis of the amount of 1:1 $CO/H_2$ consumed. The latter was calculated on the basis of the pressure drop in the 1 liter $CO/H_2$ cylinder. Reactant conversion calculated on the basis of CO consumption was plotted against the reaction time to determine the reaction rate. The reaction rate was expressed as the fraction of the theoretical $CO/H_2$ requirement consumed per minute (k $min^{-1}$). The reaction was discontinued when the reaction rate drastically dropped. Dependent on the side reaction, such as butene-1 hydrogenation and butene-1 to butene-2 isomerization, the stability of the catalyst complex in the mixture, such a rate drop occurred generally between 80—98% conversion. Accordingly, the reactions were usually discontinued in that conversion range. Most often the reactions were run up to 80% conversion.

When the reaction was to be discontinued, the $CO/H_2$ feed valve was shut and the autoclave was immediately cooled with cool water. In case of low conversions, ice bath was used. When cooling was complete, the synthesis gas was released slowly. The residual liquid was visually observed for catalyst decomposition. A dark orange to brown color of the originally yellow mixture indicated increasing degrees of catalyst decomposition. Severe catalyst decomposition usually resulted in the precipitation of dark solids.

Analyses of the residual liquid mixture were carried out using gas chromatography. The liquids were analyzed in a gc instrument using flame ionization detector. By this instrument, the $C_4$ hydrocarbons were detected. Due to the lower response of this detector to the aldehydes, the intensity of the hydrocarbon peaks was multiplied usually by 0.7 to obtain the necessary concentration correction. The individual, gaseous $C_4$ hydrocarbons were separated by another chromatograph. At first the gases were separated from the liquids and then the individual components of the gas were chromatographed and detected by a thermal conductivity detector.

1-Butene hydroformylation experiments (Example 75)

In the following description of 1-butene hydroformylation catalysis by tris-(alkyl diaryl phosphine) rhodium carbonyl hydride based catalyst systems, at first their unique catalytic behavior will be exemplified by a detailed description of the tris-(trimethylsilylethyl diphenyl phosphine) rhodium carbonyl hydride, i.e. SEP complex, plus SEP system. For comparison, detailed data will be also provided on the known tris-(triphenyl phosphine) rhodium carbonyl hydride, i.e. TPP complex, plus TPP system. This will be followed by short descriptions of the catalytic behavior of various substituted and unsubstituted alkyl diphenyl phosphines. Finally, an example of a continuous hydroformylation process based on the SEP system will be described.

Example 75

Tris-(trimethylsilylethyl diphenyl phosphine) rhodium carbonyl hydride as a catalyst in the presence of 140-fold ligand excess at different temperatures

The complex of Example 48 was studied at the 107 ppm rhodium level in the presence of 140-fold trimethylsilylethyl diphenyl phosphine (SEP) ligand as a butene hydroformylation catalyst using the general procedure. Comparative experiments were run using 107 ppm rhodium as a tris-(triphenyl phosphine) carbonyl hydride complex with 140-fold triphenyl phosphine (TPP). Reaction rates, n/i product ratios, conversions and by-products were determined at various temperatures.

The data show that both the SEP and the TPP based catalyst systems are highly active and produce a high ratio of n/i products at most temperatures. However, the temperature dependence of the two systems is very different.

The novel SEP catalyst system exhibits an increasing activity with elevated temperatures. At 100°C. and 120°C, the n/i ratio of products is about the same and there is only a small n/i drop at 145°C. High butene conversion is observed at all temperatures. The only adverse effect of temperature increase is the increased hydrogenation and isomerization of the butene-1 reactant. The SEP system remains clear, bright yellow in appearance, even at 145°C.

The known TPP catalyst system exhibits the same increased activity at 120°C. and 140°C. However, the n/i ratios in this case are dramatically reduced with increasing temperatures. At 145°C., the n/i ratio products is significantly lower in the TPP than in the SEP system. At 145°C., the reaction rate of the TPP system also drops. Decomposition of this system at this temperature is indicated by darkening of the reaction mixture. The behavior of the SEP and TPP systems is compared by Figures 5A and 5B.

The results of similar but more extensive studies are shown by Figures 6 to 9. Figure 6 correlates the hydroformylation rate with the temperature. It shows that in the presence of the SEP complex catalyst, the rates of 1-butene hydroformylation were increasing with elevated temperatures up to 155°C. In the case of the TPP catalyst, increased rates were observed only to about 135°C. Beyond these temperatures, reduced hydroformylation rates were observed apparently due to catalyst decomposition.

Figure 7 correlates the hydroformylation temperature with the selectivity for producing the linear (n-) versus branched (i-) aldehyde. It is shown that the n/i ratios depend on the temperature in the case of both catalysts.

When hydroformylations are carried out above the stable temperature range of the catalysts, a drastic drop in the n/i ratios is observed. This drop occurs above 165°C for the SEP catalyst and above 135°C for the TPP catalyst. At the same hydroformylation temperature, the use of the SEP catalyst leads to somewhat higher n/i ratios. It appears that the SEP catalyst could be used at an about 20°C higher hydroformylation temperature than the TPP catalyst and would still exhibit a selectivity equal to that of TPP at the lower temperature.

With regard to the undesired hydrogenation of the 1-butene feed to produce n-butane (Figure 8), it is noted that, in the case of the SEP catalyst, the percentage of n-butane formed more than triples to about 11% when the reaction temperature is increased from 100°C to 135°C. However, there is very little increase between 135°C and 165°C. In contrast, limited data indicate that, in the case of the TPP catalyst, the level of hydrogenation stays around the 10% level between 100°C and 140°C.

The behavior of the SEP and TPP catalysts appears to be also different with respect to the isomerization of the 1-butene feed to cis- and trans-butene-2 by-products (Figure 9). In general, less isomerization occurs when the SEP catalyst is used. However, the percentage of isomerized olefin is increased with temperature in the presence of both catalysts, up to 165°C. When the SEP catalyst becomes unstable at 170°C, less 2-butenes by-products are obtained apparently due to secondary reactions, i.e., hydrogenation and hydroformylation. In contrast, the thermal destabilization of the TTP catalyst in the 140—145° range results in a large increase of the percentage of 2-butenes in the reaction mixture.

**Claims**

1. A catalyst or complex of the formula:

$$[(Ar_2PQ)_bE^yR_{y-b}]_g \cdot [Rh(CO)H]_s$$

wherein

Ar is an aryl group containing from 6 to 10 carbon atoms,

Q is an alkylene radical or an alkylene radical, the carbon chain of which is interrupted with at least one ether oxygen or phenylene group, wherein said alkylene radical contains from 2 to 30 carbon atoms;

E represents 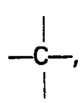, 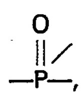, $—SO_2—$, 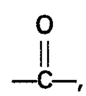, 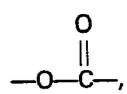, 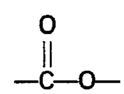 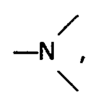,

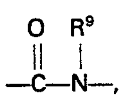, 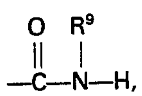, 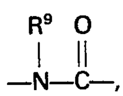, 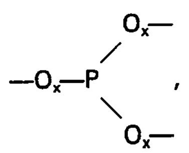, 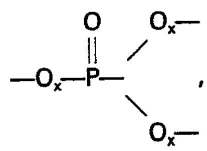, —O— or —S—,

wherein $R^9$ is H, an alkyl group containing from 1 to 30 carbon atoms or an aryl group containing from 6 to 10 carbon atoms, and wherein x is 0 or 1 with the proviso that at least one x is 1;

y represents the bonds of the group E available for bonding to said Q and R groups;

b represents an integer of from 1 to 4 provided that y-b is not less than zero;

R independently represents an alkyl group containing from 1 to 30 carbon atoms or an aryl group containing from 6 to 10 carbon atoms and with the proviso that when

E is 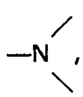, y=3, and b=1, R is $R_2$, the divalent radical

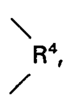, 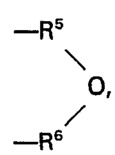, 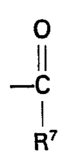 or 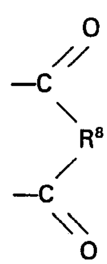

which together with the N atom form a heterocyclic ring, wherein $R^4$, $R^5$, $R^6$, $R^7$ and $R^8$ are hydrocarbyl radicals such that said heterocyclic ring contains not more than 15 atoms;

g is 1 to 6 with the proviso that g multiplied by b is 1 to 6; and

s is 1 to 3.

2. A catalyst or complex according to claim 1 wherein b is 1, g is 3 and s is 1;

$E^yR_{y-1}$ represents 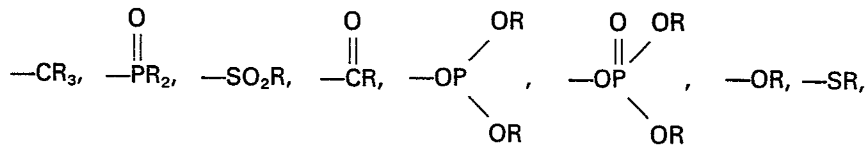

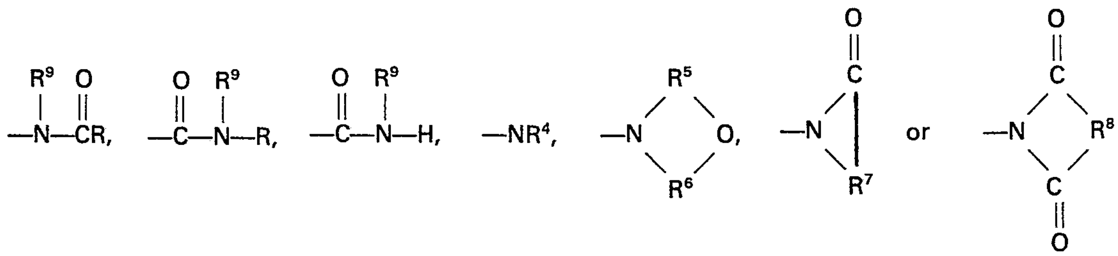

or

wherein each R independently represents an aryl group containing from 6 to 10 carbon atoms or an alkyl group containing from 1 to 30 carbon atoms, $R^4$, $R^5$, $R^6$, $R^7$ and $R^8$ are hydrocarbyl radicals such that the heterocyclic ring contains 5 or 6 carbon atoms, and $R^9$ is H, an alkyl group containing from 1 to 30 carbon atoms or an aryl group containing from 6 to 10 carbon atoms.

3. A catalyst or complex according to claim 2 of the formula

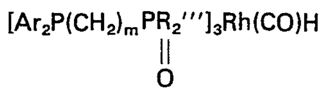

where m is 2 to 22 and R''' is a $C_1$ to $C_{30}$ hydrocarbyl radical.

4. A catalyst or complex according to claim 3 of the formula

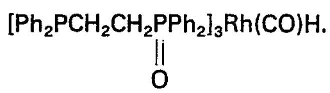

5. A rhodium-containing catalyst or complex according to claim 1 wherein b is 1, y is 3, E is N and $R_2$ is $R^4$,

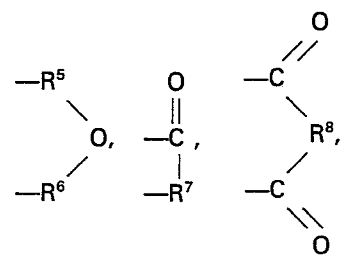

as defined in claim 1.

6. A catalyst or complex according to claim 5 having the formula:

$$[Ar_2PQ—NR_2]_3RhH(CO)$$

7. A catalyst or complex according to claim 6 having the formula:

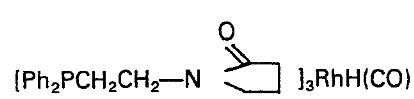

wherein Ph is phenyl.

8. A catalyst or complex of the formula:

$$[(Ar_2PQ)_ySiR_{4-y}]_g \cdot (MX_n)_s$$

wherein Ar is a substituted or unsubstituted $C_6$ to $C_{10}$ aromatic radical, Q is a substituted or unsubstituted $C_1$ to $C_{30}$ saturated open chain alkylene radical, R is an unsubstituted or mono-substituted $C_1$ to $C_{10}$ hydrocarbyl radical, M, a Group VIII transition metal is Co, Rh, Ir, Ru, Fe or Os, X is an anion or organic ligand excluding halogen, satisfying the coordination sites of the metal, y is 1 to 4, g is 1 to 6 with the proviso that g times y is 1 to 6, n is 2 to 6, and s is 1 to 3, said substituents on said aromatic radical, on said alkylene radical and on said hydrocarbyl radical being chemically stable in hydroformylation reactions, and preferably being $C_1$ to $C_{30}$ alkyl, alkoxy, aryl, acyloxy, arylamino, carbamido, carbohydrocarbyloxy, halogen, phenoxy, hydroxy or carboxy.

9. A catalyst or complex according to claim 8 wherein M is Rh, X is (CO)H; Q is an unsubstituted $C_1$ to $C_{30}$ saturated open chain alkylene radical; and R is an unsubstituted $C_1$ to $C_{10}$ hydrocarbyl radical.

10. A rhodium-containing catalyst or complex according to claim 9, having the formula:

$$\{[\phi_2P(CH_2)_m]Si(CH_3)_2\}_3[Rh(CO)H]$$

wherein m is 2 to 14 and $\phi$ is $C_6H_5$.

11. A rhodium-containing catalyst or complex according to claim 10, having the formula:

$$[(\phi_2PCH_2CH_2)_2Si(CH_3)_2]_3Rh(CO)H$$

12. Tris-(pyridylethyl diphenyl phosphine) rhodium carbonyl hydride or tris-(furylethyl diphenyl phosphine) rhodium carbonyl hydride.

13. A hydroformylation process comprising reacting an olefinic compound with $H_2$ and CO in the presence of a complex or catalyst defined in any preceding claim.

## Patentansprüche

1. Katalysator oder Komplex mit der Formel:

$$[(Ar_2PQ)_bE^yR_{y-b}]_g \cdot [Rh(CO)H]_s,$$

in der Ar eine Arylgruppe mit 6 bis 10 Kohlenstoffatomen ist, Q ein Alkylenrest oder ein Alkylenrest, dessen Kohlenstoffkette durch mindestens einen Ethersauerstoff oder eine Phenylengruppe unterbrochen ist, ist, wobei der Alkylenrest 2 bis 30 Kohlenstoffatome enthält,

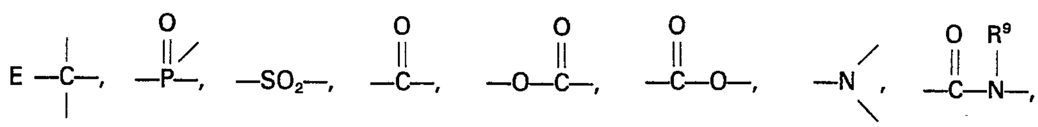

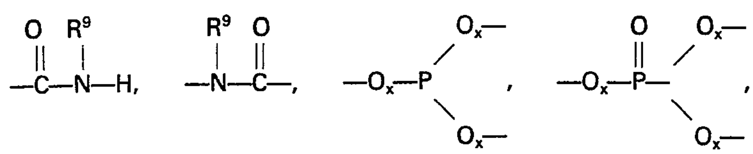

—O— oder —S— ist, wobei $R^9$ H, eine Alkylgruppe mit 1 bis 30 Kohlenstoffatomen oder eine Arylgruppe mit 6 bis 10 Kohlenstoffatomen und x O oder 1 mit der Maßgabe ist, daß mindestens ein x 1 ist, y die für die Bindung an die Q- und R-Gruppen zur Verfügung stehenden Bindungen angibt, b eine ganze Zahl von 1 bis 4 bedeutet, vorausgesetzt, daß y-b nicht weniger als 0 ist, R unabhängig eine Alkylgruppe mit 1 bis 30 Kohlenstoffatomen oder eine Arylgruppe mit 6 bis 10 Kohlenstoffatomen mit der Maßgabe bedeutet, daß, wenn

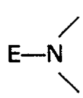 ist, y=3 ist und b=1 ist, R $R_2$, der zweiwertige Rest

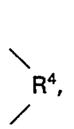, 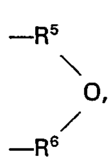, 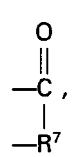, oder 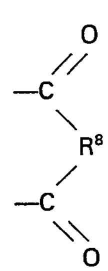

der zusammen mit dem N-Atom einen heterocyclischen Ring bildet, wobei $R^4$, $R^5$, $R^6$, $R^7$ und $R^8$ Hydrocarbylreste sind, so daß der heterocyclische Ring nicht mehr als 15 Atome enthält, ist, g 1 bis 6 mit der Maßgabe ist, daß g multipliziert mit b 1 bis 6 ist, und s 1 bis 3 ist.

2. Katalysator oder Komplex nach Anspruch 1, dadurch gekennzeichnet, daß b 1 ist, g 3 ist und s 1 ist,

$E^yR_{y-1}$ $—CR_3$, 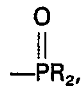, $—SO_2R$, 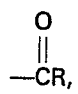, 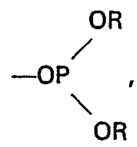, 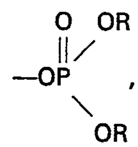, $—OR$, $—SR$, 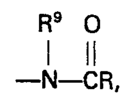,

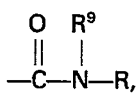, 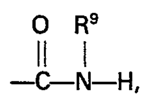, $—NR^4$, 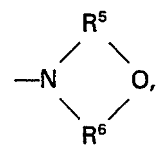, 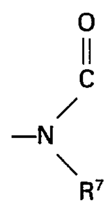 oder 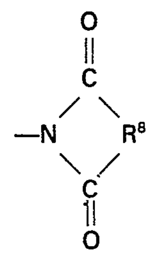

bedeutet, wobei jedes R unabhängig eine Arylgruppe mit 6 bis 10 Kohlenstoffatomen oder eine Alkylgruppe mit 1 bis 30 Kohlenstoffatomen bedeutet, $R^4$, $R^5$, $R^6$, $R^7$ und $R^8$ solche Hydrocarbylreste sind, daß der heterocyclische Ring 5 oder 6 Kohlenstoffatome enthält, und $R^9$ H, eine Alkylgruppe mit 1 bis 30 Kohlenstoffatomen oder eine Arylgruppe mit 6 bis 10 Kohlenstoffatomen ist.

3. Katalysator oder Komplex nach Anspruch 2 mit der Formel:

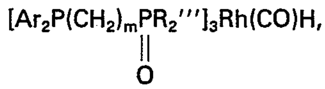

wobei m 2 bis 22 und R''' ein $C_1$—$C_{30}$-Hydrocarbylrest ist.

4. Katalysator oder Komplex nach Anspruch 3 mit der Formel:

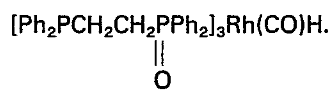

5. Rhodiumhaltiger Katalysator oder Komplex nach Anspruch 1, dadurch gekennzeichnet, daß b 1 ist, y 3 ist, E N ist und $R_2$ $R^4$,

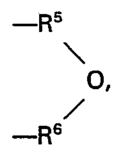 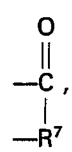, oder 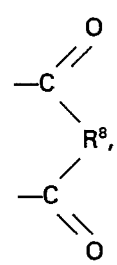

wie in Anspruch 1 definiert ist.

6. Katalysator oder Komplex nach Anspruch 5 mit der Formel:

$$[Ar_2PQ—NR_2]_3RhH(CO).$$

7. Katalysator oder Komplex nach Anspruch 6 mit der Formel:

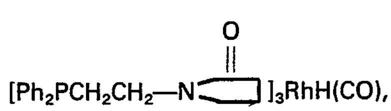

in der Ph Phenyl ist.

8. Katalysator oder Komplex mit der Formel:

$$[(Ar_2PQ)_ySiR_{4-y}]_g \cdot (MX_n)_s,$$

in der Ar ein substituierter oder nicht-substituierter aromatischer $C_6$—$C_{10}$-Rest ist, Q ein substituierter oder nicht-substituierter, gesättigter, offenkettiger $C_1$—$C_{30}$-Alkylenrest ist, R ein nicht-substituierter oder monosubstituierter $C_1$—$C_{10}$-Hydrocarbylrest ist, M, ein Gruppe VIII-Übergangsmetall, Co, Rh, Ir, Ru, Fe oder Os ist, X ein Anion oder ein organischer Ligand ausgenommen Halogen ist, das bzw. der die Koordinationsstellen des Metalls absättigt, y 1 bis 4 ist, g 1 bis 6 mit der Maßgabe ist, daß g×y 1 bis 6 ist, n 2 bis 6 ist und s 1 bis 3 ist, wobei die Substituenten am aromatischen Rest, am Alkylenrest und am Hydrocarbylrest in Hydroformylierungsreaktionen chemisch stabil sind und vorzugsweise $C_1$—$C_{30}$-Alkylreste, Alkoxyreste, Acylreste, Acyloxyreste, Acrylamidoreste, Carbamidoreste, Carbohydrocarbyloxyreste, Halogen, Phenoxyreste, Hydroxygruppen oder Carboxyreste sind.

9. Katalysator oder Komplex nach Anspruch 8, dadurch gekennzeichnet, daß M Rh ist, X (CO)H ist, Q ein nicht-substituierter, gesättigter, offenkettiger $C_1$—$C_{30}$-Alkylenrest ist und R ein nicht-substituierter $C_1$—$C_{10}$-Hydrocarbylrest ist.

10. Rhodiumhaltiger Katalysator oder Komplex nach Anspruch 9 mit der Formel:

$$\{[\phi_2P(CH_2)_m]Si(CH_3)_2\}_3[Rh(CO)H],$$

in der m 2 bis 14 ist und $\phi$ $C_6H_5$ ist.

11. Rhodiumhaltiger Katalysator oder Komplex nach Anspruch 10 mit der Formel:

$$[(\phi_2PCH_2CH_2)_2Si(CH_3)_2]_3Rh(CO)H$$

12. Tris-(Pyridylethyldiphenylphosphin)-rhodiumcarbonylhydrid oder tris-(Furylethyldiphenylphosphin)rhodiumcarbonylhydrid.

13. Hydroformylierungsverfahren, dadurch gekennzeichnet, daß eine olefinische Verbindung mit $H_2$ und CO in Gegenwart eines Komplexes oder Katalysators gemäß Definition in einem der vorangehenden Patentansprüche umgesetzt wird.

**Revendications**

1. Catalyseur ou complexe de formule

$$[(Ar_2PQ)_bE^yR_{y-b}]_g \cdot [Rh(CO)H]_s$$

dans laquelle

Ar représente un groupe aryle de 6 à 10 atomes de carbone

Q représente un radical alkylène ou un radical alkylène dont la chaîne carbonée est interrompue par au moins un groupe éther-oxyde ou phénylène, ledit radical alkylène comportant de 2 à 30 atomes de carbone

E représente 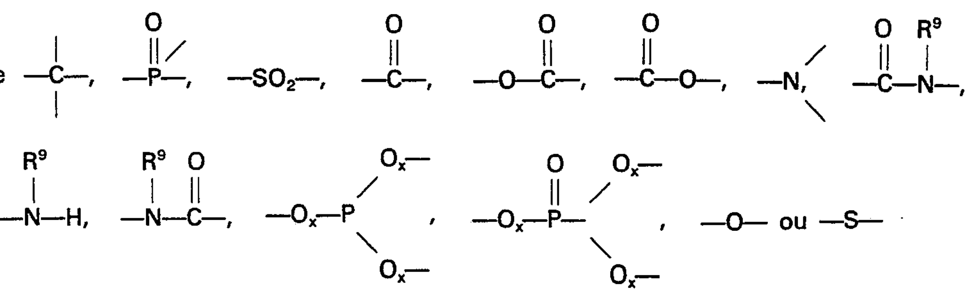

tel que $R^9$ représente H, un groupe alkyle de 1 à 30 atomes de carbone ou un groupe aryle de 6 à 10 atomes de carbone, et x soit 0 ou 1 à la condition que au moins l'un des x soit 1;

y représente les liaisons du groupe E qui peuvent se fixer aux groupes Q et R

b représente un nombre entier de 1 à 4, de telle sorte que y-b ne soit pas inférieur à zéro

R représente indépendamment un groupe alkyle contenant de 1 à 30 atomes de carbone ou un groupe aryle contenant de 6 à 10 atomes de carbone, de telle sorte que lorsque

E représente— 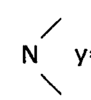 y=3 et b=1, R est $R_2$, le radical divalent

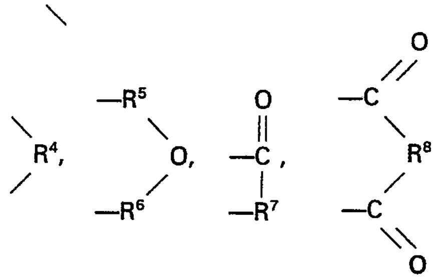

qui avec l'atome d'azote forme un hétérocycle, tel que $R^4$, $R^5$, $R^6$, $R^7$ et $R^8$ représentent des radicaux hydrocarbyles de telle sorte que cet hétérocycle ne comporte pas plus de 15 atomes;

g représente 1 à 6, à la condition que g multiplié par b soit 1 à 6, et représente 1 à 3.

2. Catalyseur ou complexe selon la revendication 1 dans lequel b est 1, g est 3 et s est 1;

$E^yR_{y-1}$ représente 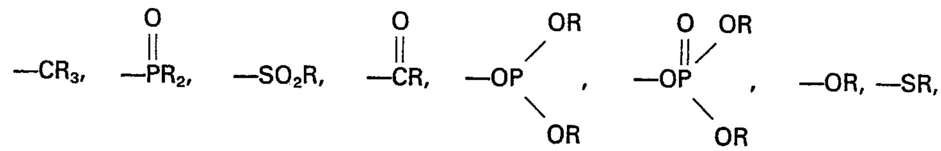

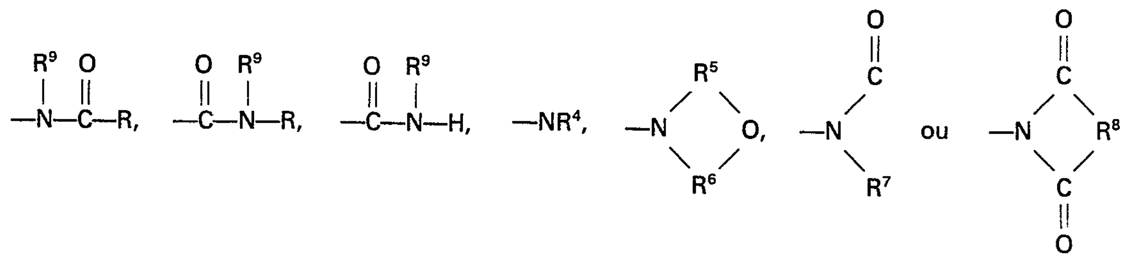

tel que chacun des R représente indépendamment un groupe aryle de 6 à 10 atomes de carbone ou un groupe alkyle de 1 à 30 atomes de carbone; $R^4$, $R^5$, $R^6$, $R^7$ et $R^8$ représentent des radicaux hydrocarbyles de telle sorte que l'hétérocycle contienne 5 ou 6 atomes de carbone et $R^9$ représente H, un groupe alkyle de 1 à 30 atomes de carbone ou un groupe aryle de 6 à 10 atomes de carbone.

3. Catalyseur ou complexe selon la revendication 2 de formule

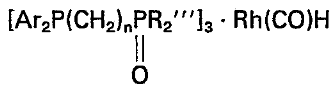

dans laquelle n est 2 à 22 et R''' est un radical hydrocarbyle en $C_1$ à $C_{30}$.

4. Catalyseur ou complexe selon la revendication 3 de formule

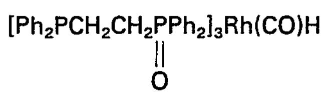

5. Catalyseur ou complexe à base de rhodium selon la revendication 1 dans laquelle b représente 1, y représente 3, E représente N et $R_2$ représente

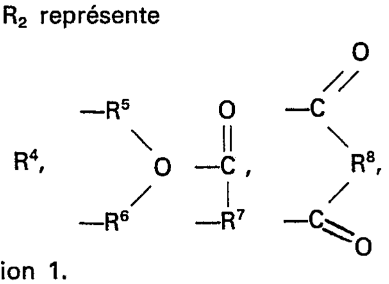

comme défini dans la revendication 1.

6. Catalyseur ou complexe selon la revendication 5 de formule

$$[Ar_2PQ—NR_2]_3RhH(CO)$$

7. Catalyseur ou complexe selon la revendication 6 de formule

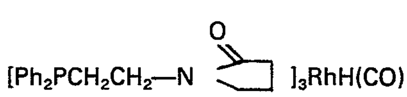

dans laquelle Ph est le phényle.

8. Catalyseur ou complexe de formule

$$[(Ar_2PQ)_ySiR_{4-y}]_g \cdot (MX_n)_s$$

dans laquelle Ar est un radical aromatique en $C_6$ à $C_{10}$ substitué ou non, Q est un radical alkylène saturé, en chaîne ouverte, de 1 à 30 atomes de carbone, substitué ou non, R est un radical hydrocarbyle en $C_1$ à $C_{10}$

substitué ou non, M, métal de transition du groupe VIII représente Co, Rh, Ir, Ru, Fe, ou Os, X représente un anion ou un ligand organique à l'exclusion d'un halogène, remplissant les sites de coordination du métal, y représente 1 à 4, g représente 1 à 6 de telle sorte que g fois y soit 1 à 6, n représente 2 à 6 et s représente 1 à 3, les substituants du radical aromatique, alkylène et hydrocarbyle étant chimiquement stables dans une réaction d'hydroformylation et étant de préférence, de $C_1$ à $C_{30}$ alkyl, alkoxy, acyl, acyloxy, acrylamido, carbamido, carbohydrocarbyloxyhalogen, phénoxy, hydroxy ou carboxy.

9. Catalyseur ou complexe selon la revendication 8 dans lequel M est Rh, X est (CO)H; Q est un radical alkylène saturé en chaîne ouverte de $C_1$ à $C_{30}$, non substitué; et R est un radical hydrocarbyle en $C_1$ à $C_{10}$ non substitué.

10. Catalyseur ou complexe à base de rhodium selon la revendication 9 de formule

$$\{[\phi_2P(CH_2)_m]Si(CH_3)_2\}_3[Rh(CO)H]$$

dans laquelle m représente 2 à 14 et $\phi$ représente $C_6H_5$.

11. Catalyseur ou complexe selon la revendication 10 de formule:

$$[\phi_2{-}PCH_2CH_2)_2Si(CH_3)_2]_3Rh(CO)H$$

12. Tris-(pyridyléthyldiphényl-phosphine) rhodium carbonylhydrure ou tris-(furyléthyldiphényl-phosphine)rhodium carbonylhydrure.

13. Procédé d'hydroformylation comportant la réaction d'un dérivé oléfinique avec $H_2$ et CO en présence d'un complexe ou d'un catalyseur défini dans l'une quelconque des revendications précédentes.

KEY STEPS IN THE MECHANISM OF PHOSPHINE-RHODIUM COMPLEX CATALYZED HYDROFORMYLATION OF OLEFINS

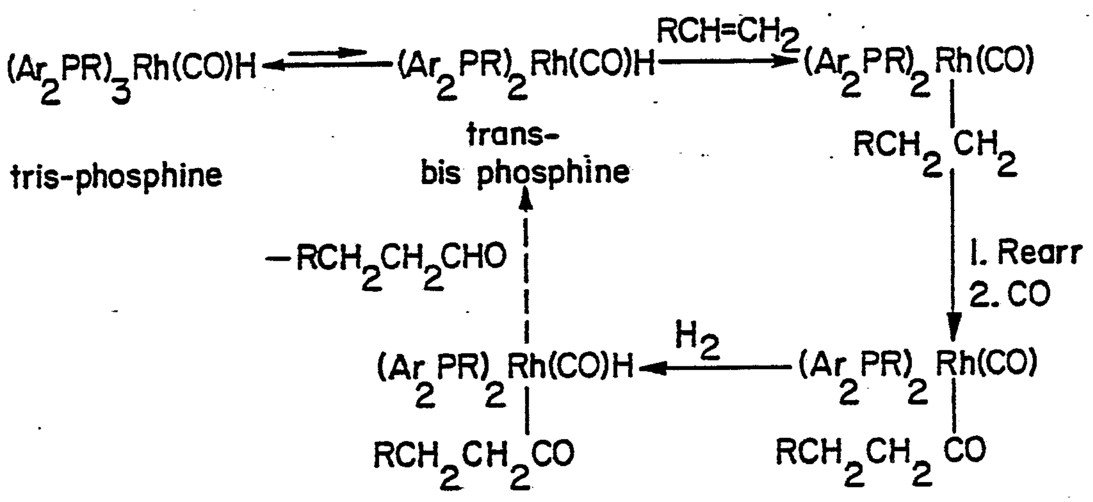


FIG.1

COMPARATIVE $^{31}P$ NMR SPECTRA OF RHODIUM COMPLEXES OF VARYING ALIPHATIC CHARACTER

Derived Via Ligand Exchange

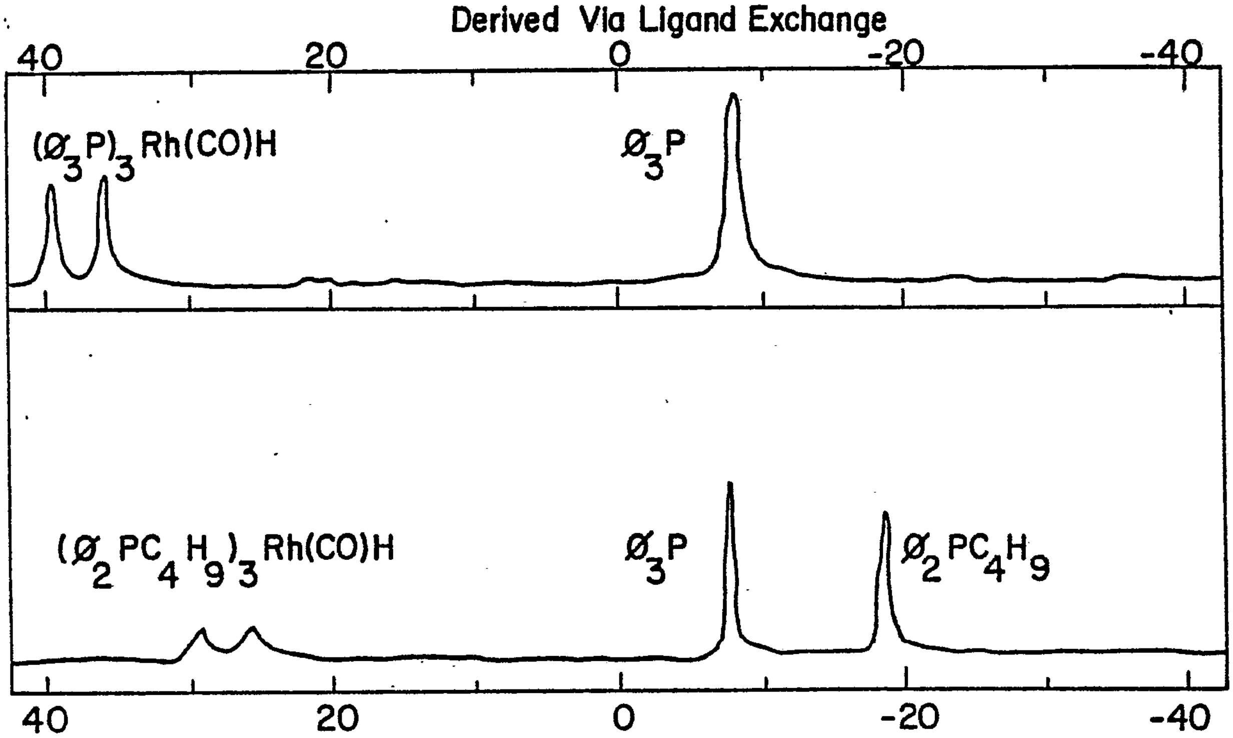


CHEMICAL SHIFT, PPM

FIG. 2

$^{31}P$ NMR STUDY OF LIGAND EXCHANGE RATES AS A FUNCTION OF TEMPERATURE

$(\text{Ø}_3P)_3Rh(CO)H + 3\text{Ø}_3P^* \rightleftharpoons (\text{Ø}_3P^*)_3Rh(CO)H + 3\text{Ø}_3P$

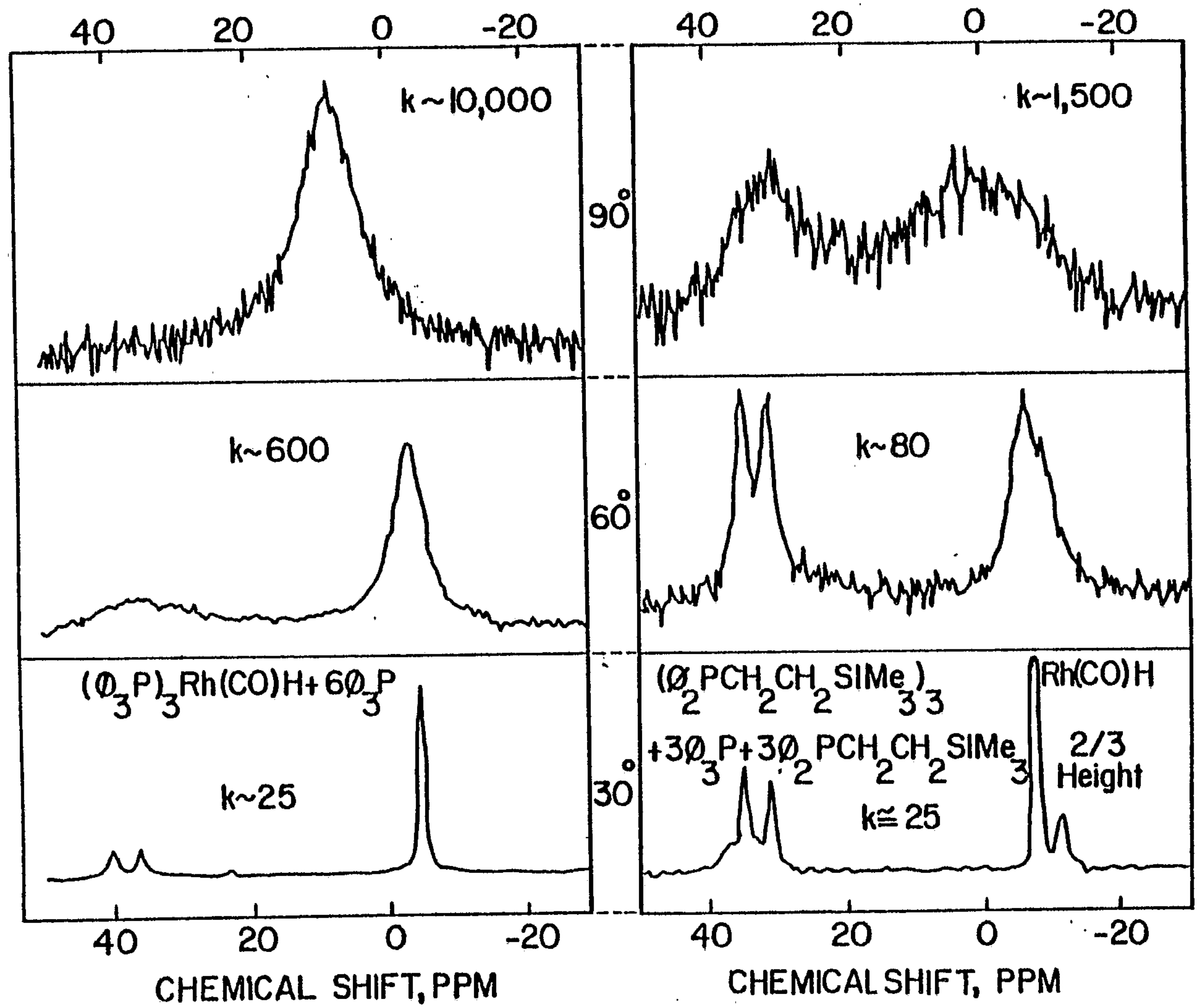


FIG.3

SCHEME OF AUTOCLAVE FOR HYDROFORMYLATION

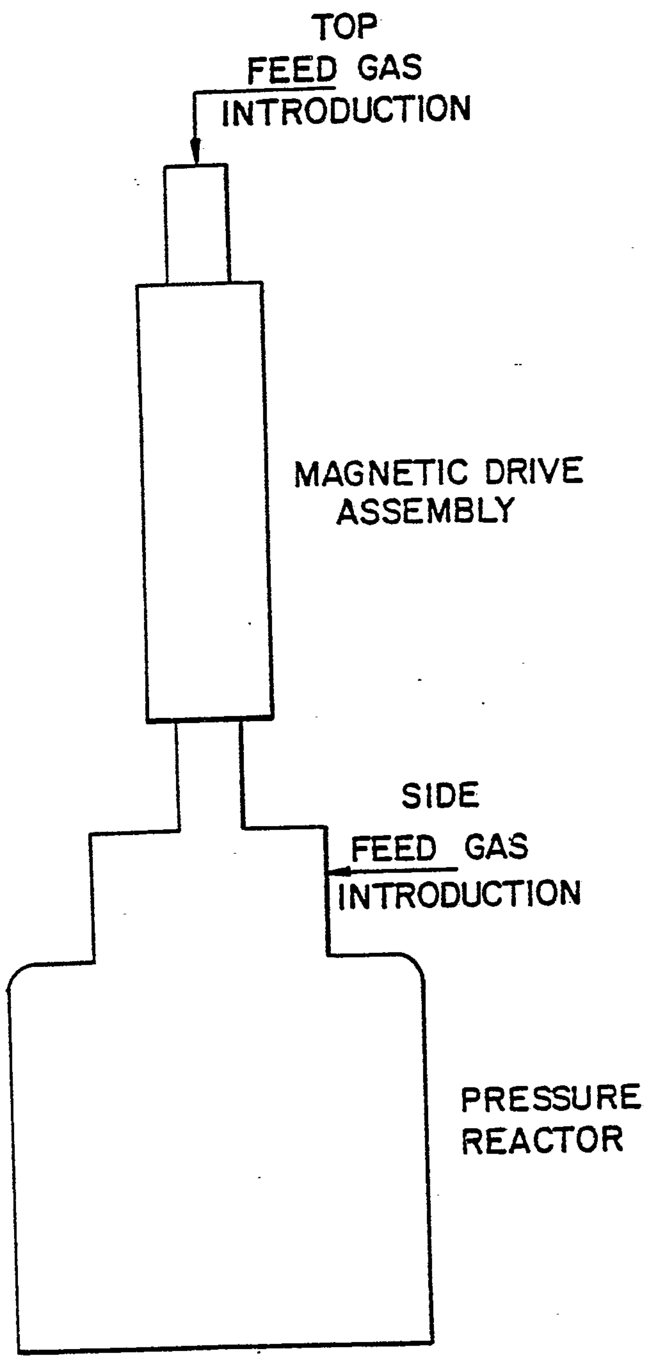


FIG. 4

CATALYST STABILITY AT HIGH TEMPERATURE
HYDROFORMYLATION OF BUTENE-1

350 psi $CO/H_2$ (1:4) AT 145°
107 ppm Rh, LIGAND/Rh = 140

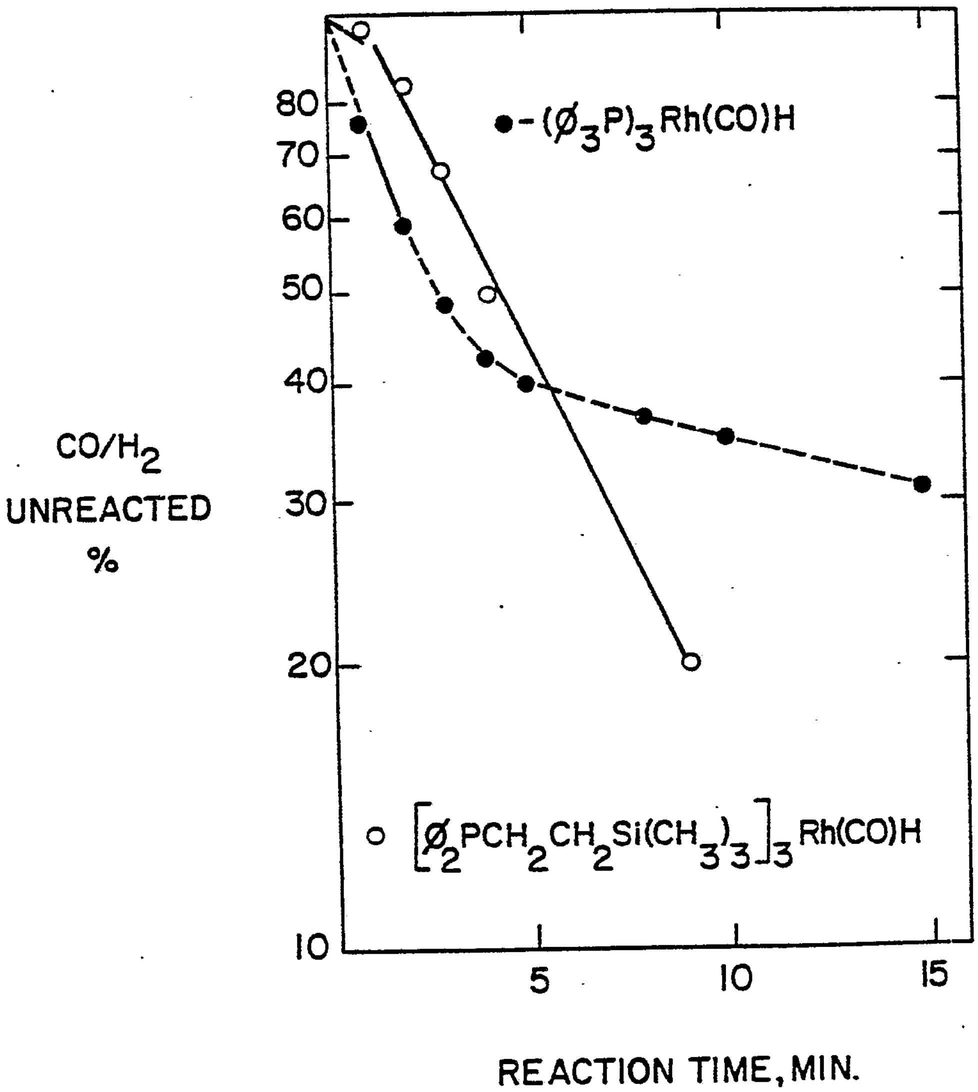


FIG. 5A

CATALYST STABILITY AT LOW LIGAND/Rh RATIO: HYDROFORMYLATION OF BUTENE-1

350psi $CO/H_2$ (1:4) AT 100°

105ppm Rh, LIGAND/Rh ≅ 27

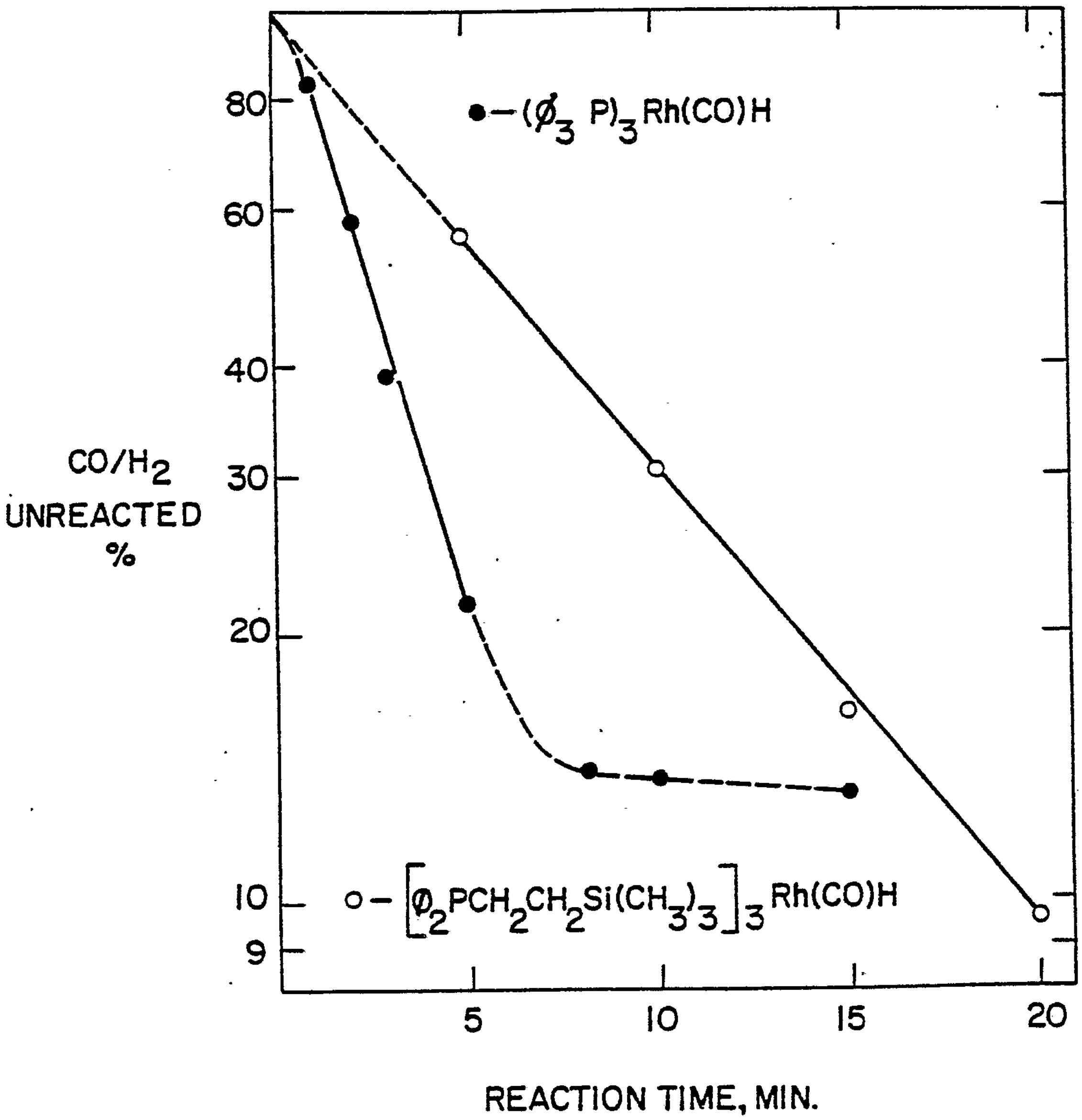


FIG. 5B

BUTENE HYDROFORMYLATION RATE VERSUS TEMPERATURE CORRELATIONS IN THE PRESENCE OF EXXON AND UNION CARBIDE RHODIUM-PHOSPHINE COMPLEX CATALYSTS

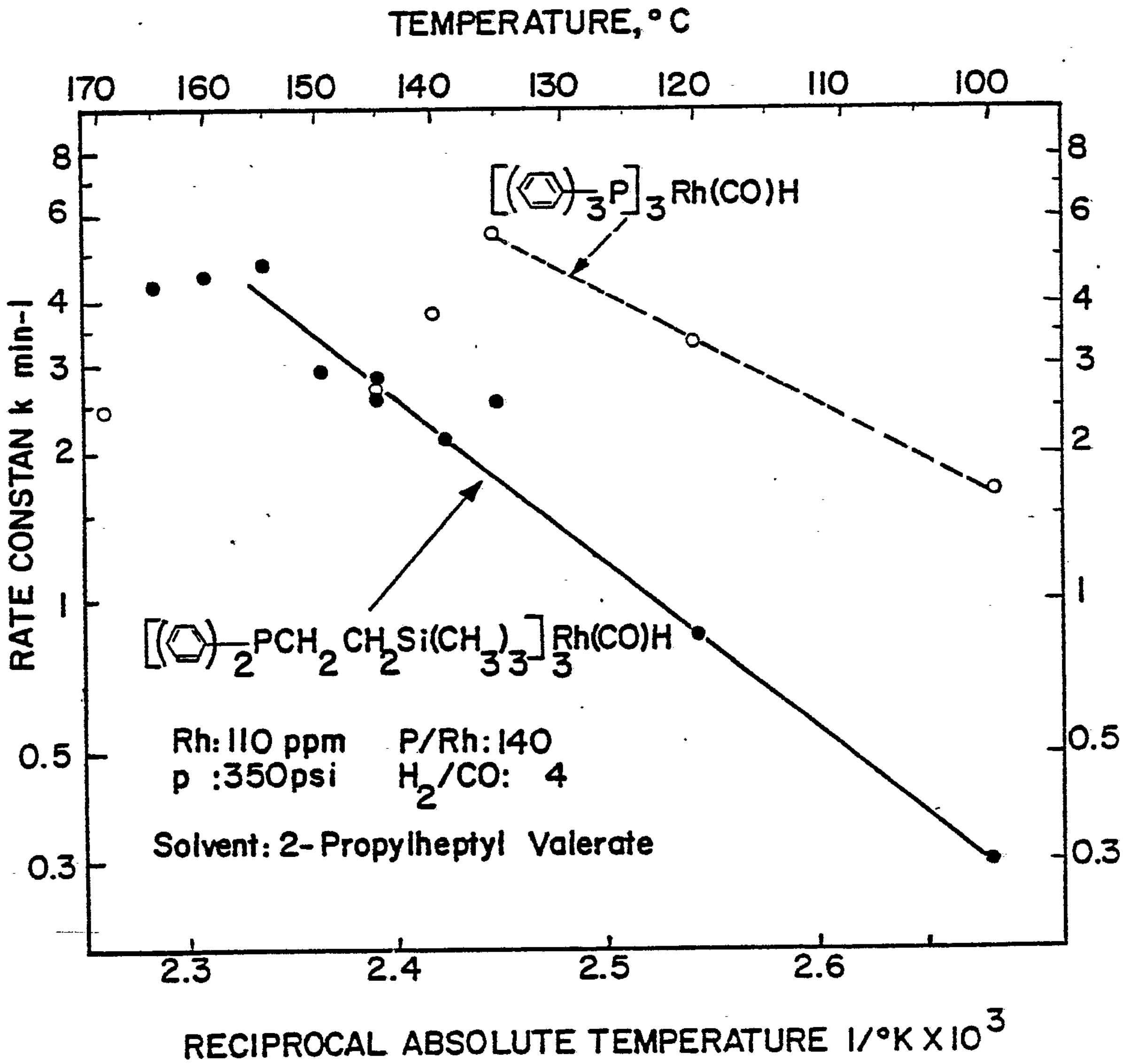


FIG.6

I-BUTENE HYDROFORMYLATION TEMPERATURE VERSUS
n-VALERALDEHYDE (n) TO TOTAL VALERALDEHYDE (n+i)
RATIO CORRELATIONS IN THE PRESENCE OF SEP AND TPP
BASED RHODIUM-PHOSPHINE COMPLEX CATALYSTS

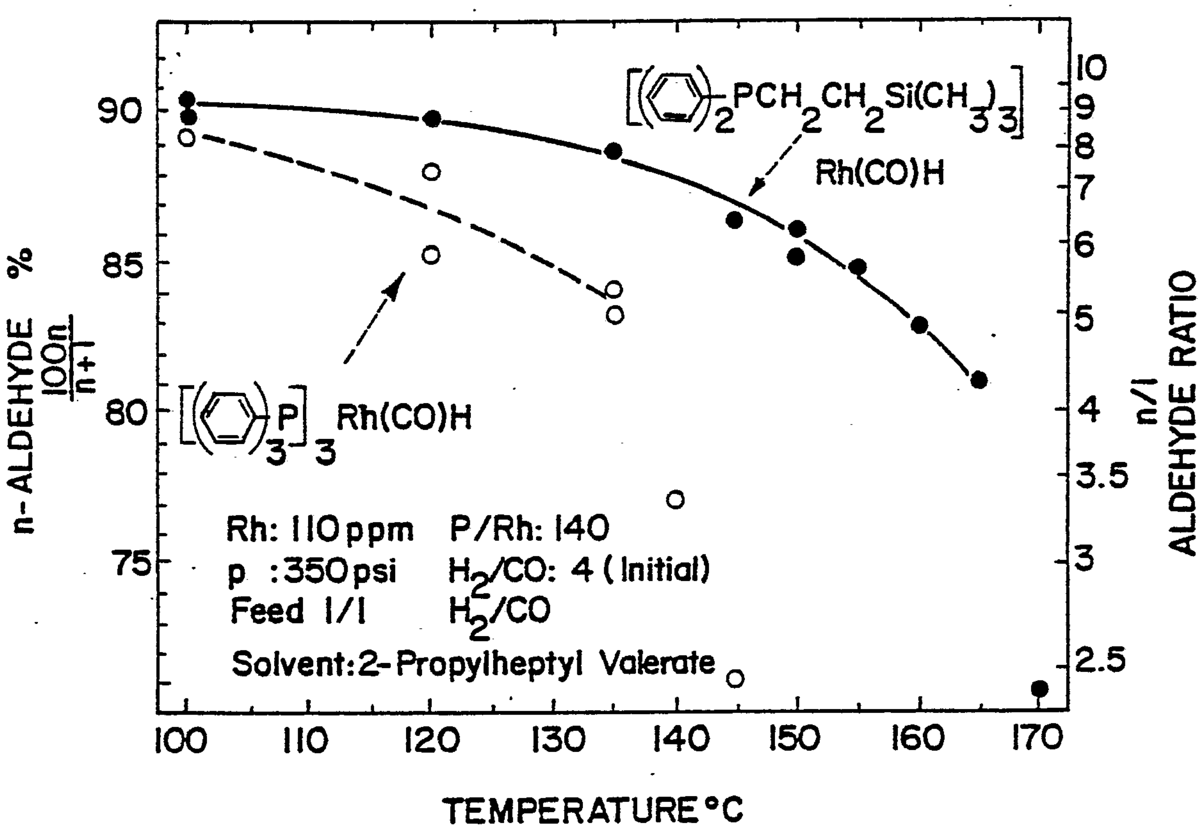


FIG. 7

I-BUTENE HYDROFORMYLATION TEMPERATURE VERSUS
BUTANE BY-PRODUCT FORMATION CORRELATIONS
IN THE PRESENCE OF SEP AND TPP BASED COMPLEX CATALYSTS

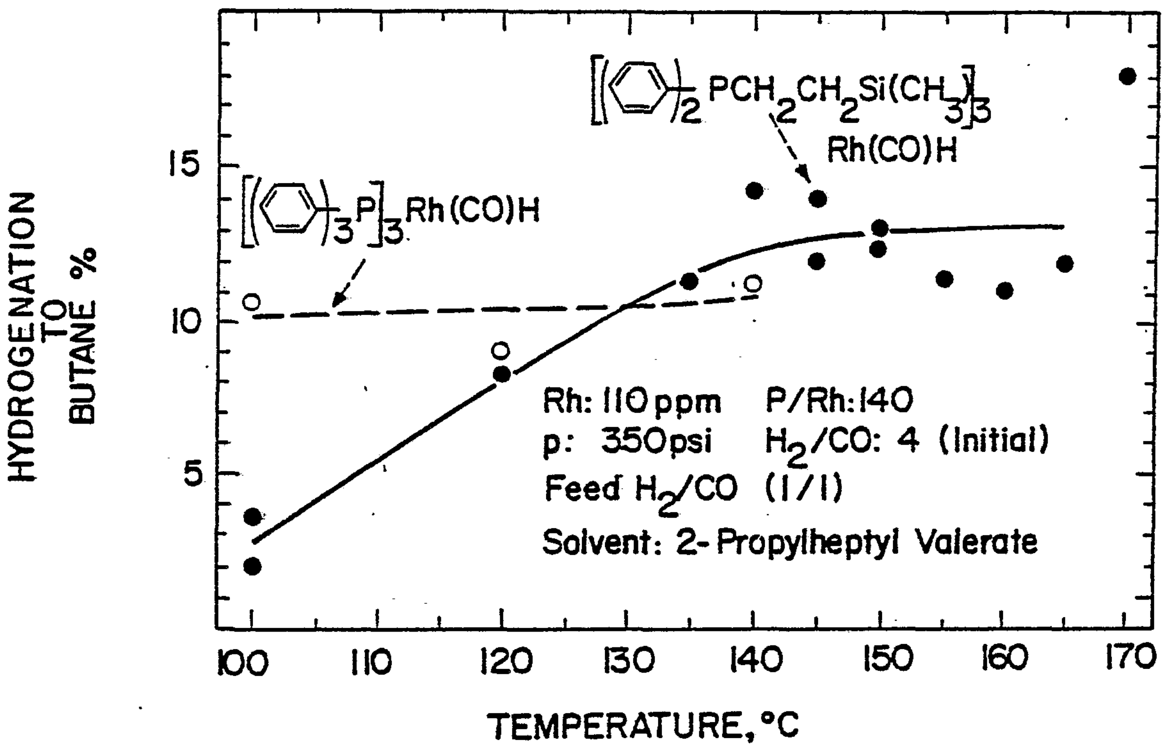


FIG. 8

1-BUTENE HYDROFORMYLATION TEMPERATURE VERSUS 2-BUTENES BY-PRODUCT FORMATION CORRELATIONS IN THE PRESENCE OF SEP AND TPP COMPLEX CATALYSTS

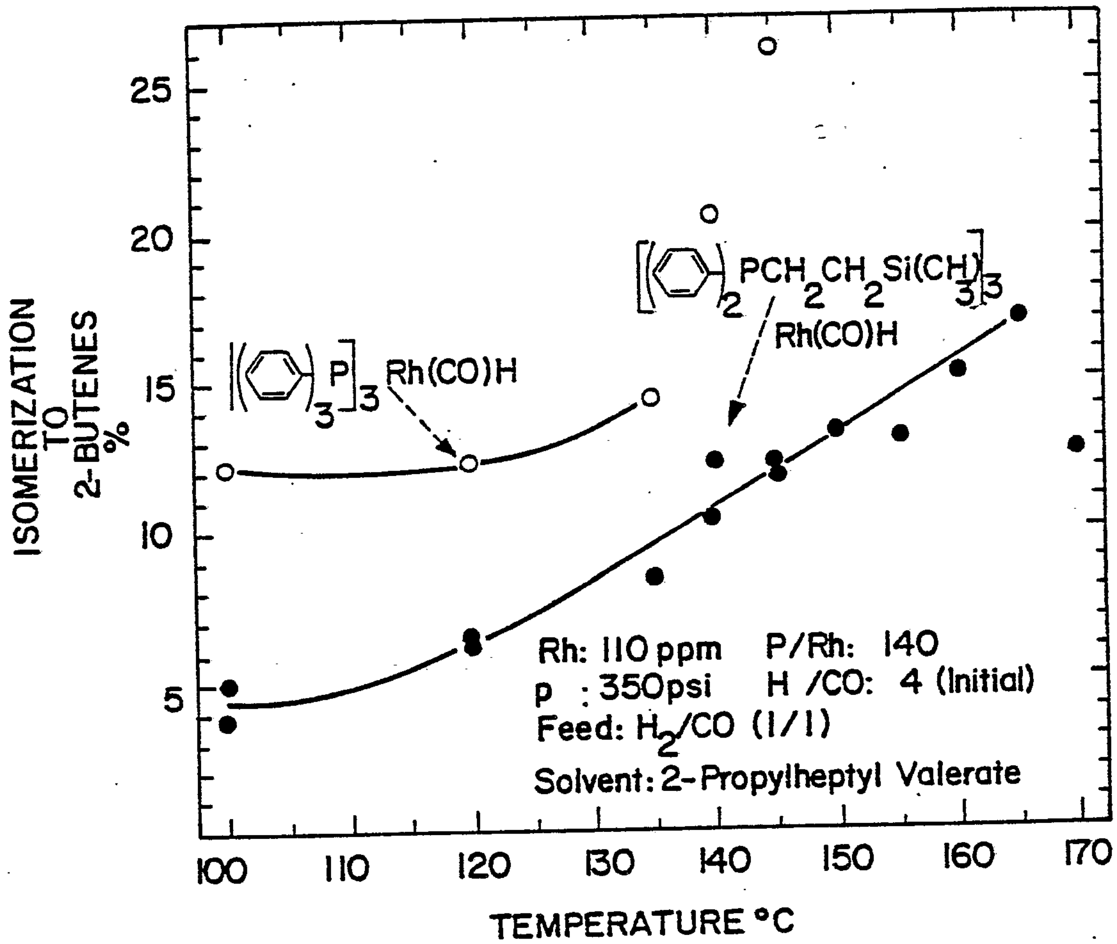


FIG. 9

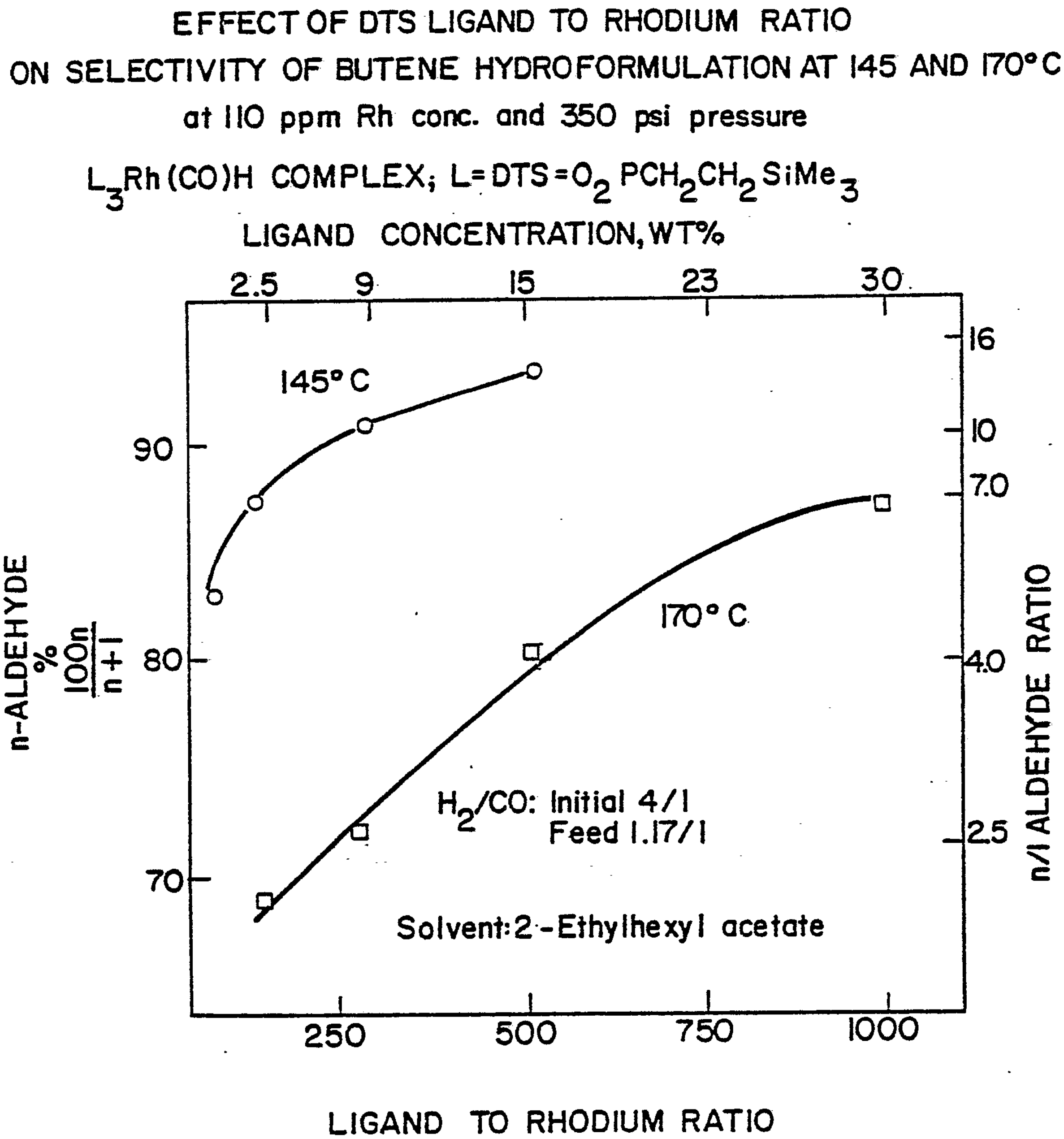
EFFECT OF DTS LIGAND TO RHODIUM RATIO
ON SELECTIVITY OF BUTENE HYDROFORMULATION AT 145 AND 170°C
at 110 ppm Rh conc. and 350 psi pressure
$L_3Rh(CO)H$ COMPLEX; $L=DTS=O_2PCH_2CH_2SiMe_3$
LIGAND CONCENTRATION, WT%
2.5
9
15
23
30
145° C
170° C
n-ALDEHYDE % $\frac{100n}{n+1}$
90
80
70
n/I ALDEHYDE RATIO
16
10
7.0
4.0
2.5
$H_2$/CO: Initial 4/1
Feed 1.17/1
Solvent: 2-Ethylhexyl acetate
250
500
750
1000
LIGAND TO RHODIUM RATIO

FIG.10

EFFECT OF CARBON MONOXIDE PRESSURE ON RATIO OF n-VALERALDEHYDE (n) TO TOTAL VALERALDEHYDE (n+I) PRODUCT OF I-BUTENE HYDROFORMYLATION

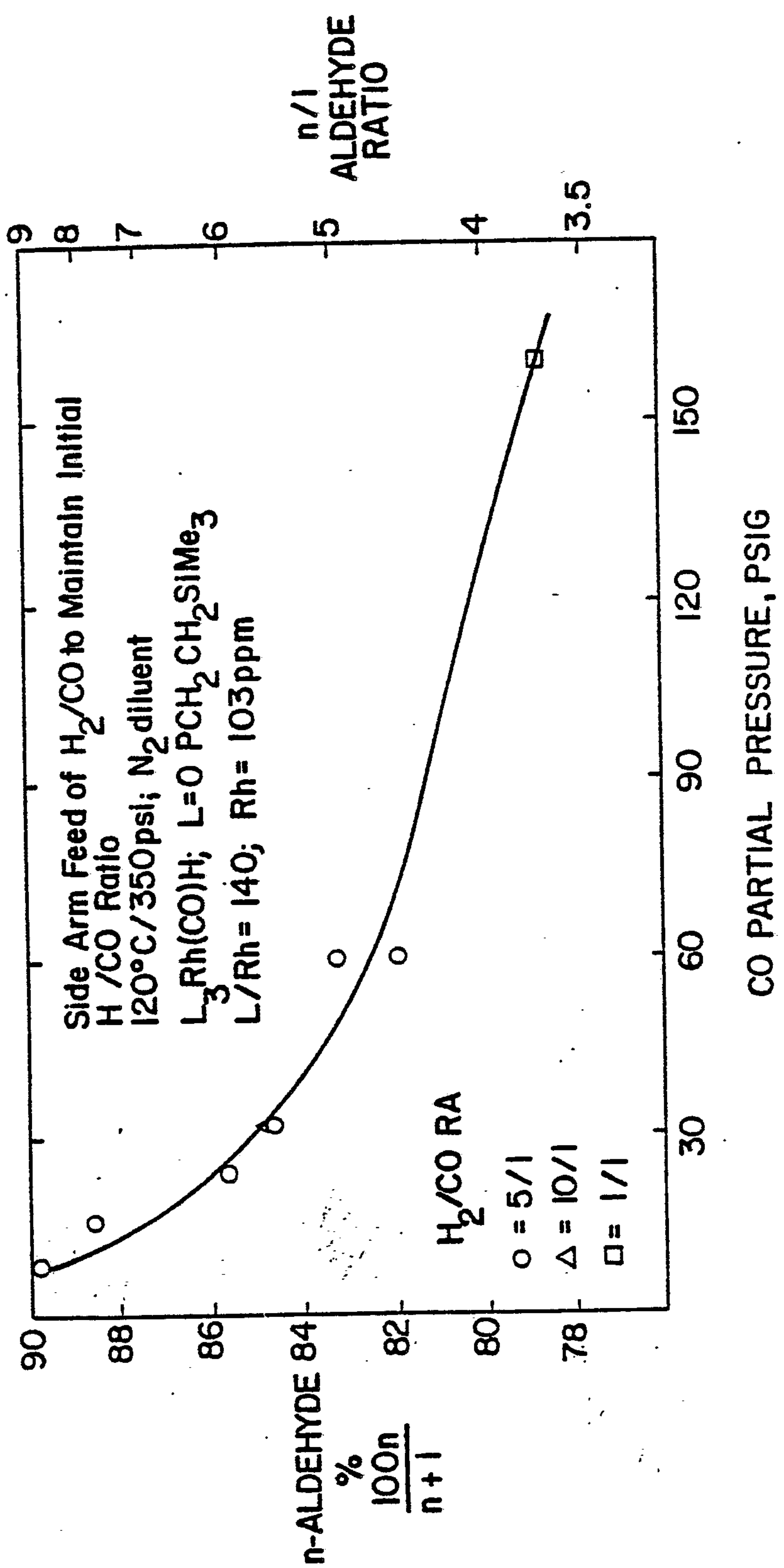


FIG.II

ALDEHYDE PRODUCTION RATE DURING CONTINUOUS BUTENE HYDROFORMYLATION
As shown with 140 psi of 10/1 $H_2$/CO at 120° for 448 hours and then at 125°
in the presence of 270 ppm Rh and Rh/P mole ratio of 210

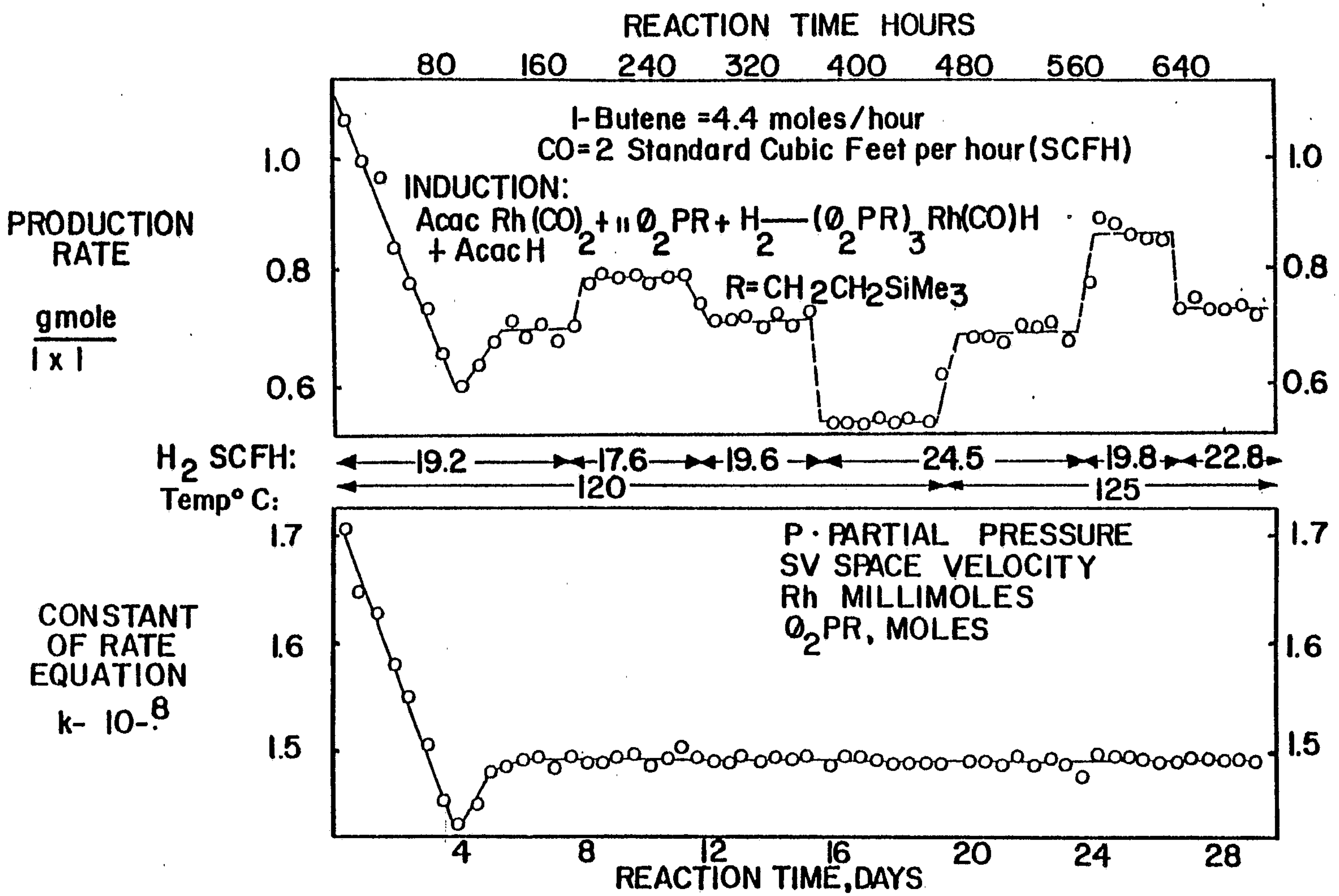


FIG.12